# EUROPEAN PATENT APPLICATION

(11) **EP 1 435 389 A1**
(43) Date of publication of application: **07.07.2004**
(21) Application number: 02762944.3
(22) Date of filing: 02.09.2002
(51) Int. Cl.: C12N 15/00, C07K 14/47, C07H 21/02, A61P 35/00, A61K 47/48

(54) **METHOD OF EVALUATING EFFICACY OF NUCLEIC ACID THERAPY**

(30) Priority: 04.09.2001 JP 2001267385
(71) Applicant: Nippon Shinyaku Co., Ltd., Kyoto-shi Kyoto 601-8550 (JP)
(72) Inventor: HIRABAYASHI, Kazuko, Kyoto-shi, Kyoto 616-8105 (JP); YANO, Junichi, Nara-shi, Nara 630-8105 (JP)
(74) Representative: VOSSIUS & PARTNER
(86) International application number: PCT/JP2002/008856
(87) International publication number: WO 2003/023031

(57) **Abstract**

A method of preliminarily evaluating the efficacy of a treatment with a nucleotide or a nucleoside having an antitumor or antiviral effect on individual patients as a pre-treatment for tailar-made therapy; pharmaceutical compositions for the evaluation; a screening method for finding out a drug having an antitumor or antiviral effect; a method of searching for a function mechanism of an antitumor agent or an antiviral agent in vivo; etc. Namely, use is made as an indication of a change in the expression dose or activity of a protein relating mainly to any one of the sequences represented by SEQ ID NO: 1-4 before and after administration of the nucleotide and nucleoside.

## Description

### TECHNICAL FIELD

The present invention is related to a method of examining efficacy of treatment with an antitumor- or antivirus agent in individual patients. The present invention is also related to a method of screening for a candidate antitumor or antivirus agent.

### BACKGROUND OF THE INVENTION

For example, synthetic nucleic acid polymers typified by polyinosinic - polycytidylic acid (i.e., polyinosinic acid · polycytidylic acid) have excellent antitumor and/or antivirus activity. Such a synthetic nucleic acid polymer has been reported that, when administered into a living body as a complex with an appropriate cationic liposome carrier, it attacks specifically cancer cells and activates an intracellular nuclease thereby leads the cells into apoptosis and that it has prominent antivirus effect on hepatitis virus (WO99/20283, WO99/48531)

However, the reactivity to antitumor agent administered differs from one patient to another, irrespective of the high potency of the drug administered, and an agent having sufficient effects in a patient while shows only poor effect in another.

Recently, tailor-made therapy, which is a method of treatment adapted to individual patients, has been proposed. The tailor-made therapy wherein treatment is conducted while confirming the efficacy or predicting side effects of a drug is a promising new therapeutic system.

### SUMMARY OF THE INVENTION

The present invention provides a method of examining efficacy (effectiveness) of treatment with antitumor- or antivirus agent in individual patients or pharmaceutical compositions for the examination, which is mainly connected to tailor-made therapy. The present invention also provides a method of screening for agents potentially having antitumor or antivirus activity.

The present inventors have intensively studied and found that there exist in a living body proteins capable of binding to or induced to express by a double-stranded RNA, which is polyinosinic - polycytidylic acid known to be an excellent antitumor or antivirus agent, and established the present invention. In the present application, three proteins of the former type and one protein of the latter type will be disclosed.

The present invention, for example, includes the following embodiments.
(1) A method of examining efficacy of treatment with a nucleotide or a nucleoside in a given subject comprising:
   (a) measuring the amount of expression or activity of at least one protein among those each having amino acid sequences of SEQ ID NO: 1-4 in a blood or tissue sample previously taken from the subject;
   (b) administering the nucleotide or the nucleoside into a tissue of the subject or a tissue taken from the subject followed by measuring the amount of expression or activity of the same protein as that measured in (a) in blood or treated tissue after 3-48 hours from administration; and
   (c) comparing the measurement obtained in step (a) with that obtained in step (b) and evaluating the change in the amount of expression or the activity of the protein.
(2) A method of examining efficacy of treatment with a nucleotide or a nucleoside in a given subject comprising:
   (a) measuring the amount of expression or activity of at least one protein among those each having amino acid sequences of SEQ ID NO: 5-30 in a blood or tissue sample previously taken from the subject;
   (b) administering the nucleotide or the nucleoside into a tissue of the subject or a tissue taken from the subject followed by measuring the amount of expression or activity of the same protein as that measured in (a) in blood or treated tissue after 3-48 hours from administration; and
   (c) comparing the measurement obtained in step (a) with that obtained in step (b) and evaluating the change in the amount of expression or the activity of the protein.
(3) A pharmaceutical composition for examining efficacy of treatment with a nucleotide or a nucleoside in a given subject comprising:
   (a) measuring the amount of expression or activity of at least one protein among those each having amino acid sequences of SEQ ID NO: 1-4 in a blood or tissue sample previously taken from the subject;
   (b) administering the nucleotide or the nucleoside into a tissue of the subject or a tissue taken from the subject followed by measuring the amount of expression or activity of the same protein as that measured in (a) in blood or treated tissue after 3-48 hours from administration; and
   (c) comparing the measurement obtained in step (a) with that obtained in step (b) and evaluating the change in the amount of expression or the activity of the protein.
(4) A pharmaceutical composition for examining efficacy of treatment with a nucleotide or a nucleoside in a given subject comprising:
   (a) measuring the amount of expression or activity of at least one protein among those each having amino acid sequences of SEQ ID NO: 5-30 in a blood or tissue sample previously taken from the subject;
   (b) administering the nucleotide or the nucleoside into a tissue of the subject or a tissue taken from the subject followed by measuring the amount of expression or activity of the same protein as that measured in (a) in blood or treated tissue after 3-48 hours from administration; and
   (c) comparing the measurement obtained in step (a) with that obtained in step (b) and evaluating the change in the amount of expression or the activity of the protein.
(5) A method of screening for agents potentially having antitumor or antivirus activity comprising examining a test compound for ability to bind to at least one protein among those each having amino acid sequences of SEQ ID NO: 1-4.
(6) A method of screening for agents potentially having antitumor or antivirus activity comprising examining a test compound for ability to bind to at least one protein among those each having amino acid sequences of SEQ ID NO: 5-30.
(7) A method of investigating the mechanism of action of an antitumor or antivirus agent *in vivo* comprising examining the antitumor or antivirus agent for ability to bind to at least one protein among those each having amino acid sequences of SEQ ID NO: 1-4.
(8) A method of investigating the mechanism of action of an antitumor or antivirus agent *in vivo* comprising examining the antitumor or antivirus agent for ability to bind to at least one protein among those each having amino acid sequences of SEQ ID NO: 5-30.

### BRIEF DESCRIPTION OF THE DRAWINGS

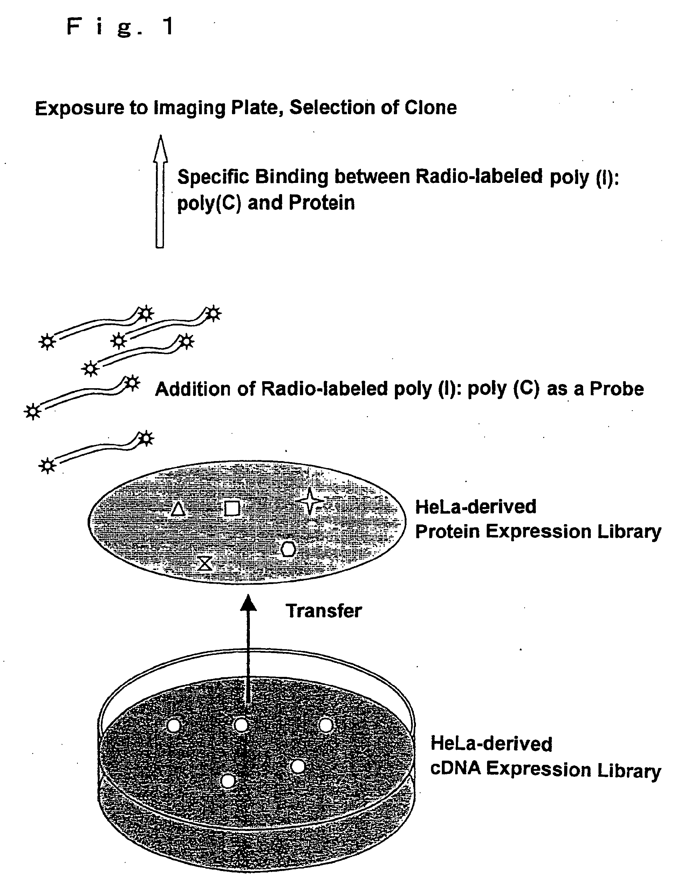

Fig. 1 is a schematic diagram of the process for detecting proteins each having amino acid sequences of SEQ ID NO: 1-3 described in Experiment 1.

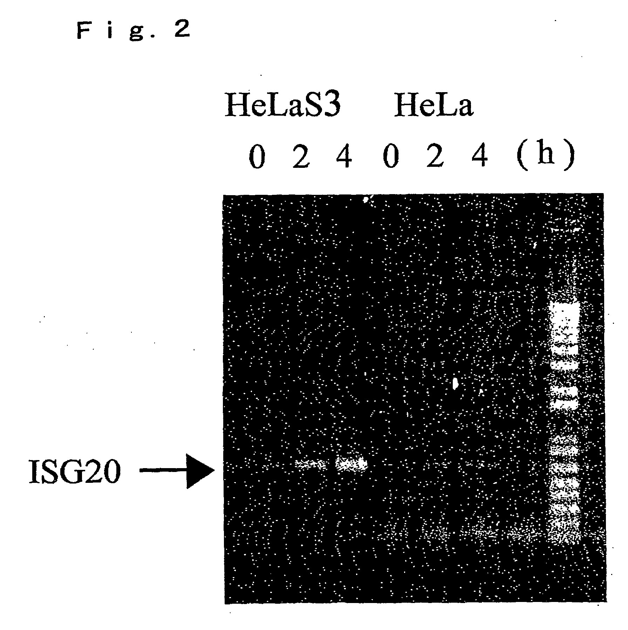

Fig. 2 is an electrophoretogram showing expression of mRNA for ISG20 in carcinoma cells, HeLaS3 (left lane) and HeLa (right lane) following the addition of a complex comprising polyinosinic - polycytidylic acid. The figures at the upper side indicate the time (hour) after administration of the complex. The bands at the rightmost lane indicate markers. The white arrow indicates the site where mRNA for ISG20 is expressed.

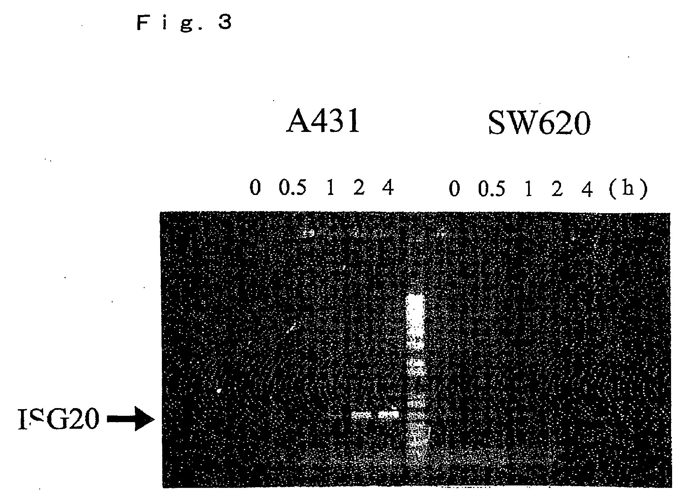

Fig. 3 is an electrophoretogram showing expression of mRNA for ISG20 in carcinoma cells, A431 (left lane) and SW620 (right lane) following the addition of a complex comprising polyinosinic - polycytidylic acid. The figures at the upper side indicate the time (hour) after administration of the complex. The bands at the middle lane indicate markers. The white arrow indicates the site where mRNA for ISG20 is expressed.

### DETAILED DESCRIPTION OF THE PREFERRED EMBODIMENTS

Nucleotides or nucleosides of which antitumor or antivirus activity is evaluated by the present method are those that exert activity when entered into a cell.

Such a nucleotide or nucleoside is, for example, administered into a tissue as an aqueous solution containing the same as it is, or as a pharmaceutical composition containing the same in association with an appropriate carrier. The proteins usable in the present method can be obtained, for example, according to the screening method illustrated hereinafter in the Experimental Examples.

### I. Proteins

Examples of proteins usable in the present method include those each having amino acid sequences of SEQ ID NO: 1-30.

The proteins having amino acid sequences of SEQ ID NO: 1-4 are all known proteins.

The protein of SEQ ID NO: 1 exists in a living body and comprises 490 amino acids. A gene encoding the protein is deposited at GenBank under accession number AK002169 (gene name: AK002169). This protein is known to comprise motifs of ribonuclease III family, receptor tyrosine kinase class III, receptor tyrosine kinase class V, dsRNA binding motif, and the like. Since the motifs of nuclease and ribonuclease exist, the protein is considered to induce apoptosis in cells when activated.

The proteins of SEQ ID NO: 5-16 are all known proteins, of which amino acid sequences are highly identical with the amino acid sequence of the protein of SEQ ID NO: 1 wherein the sequence identity is 60 % or more, and considered to have activity equivalent to that of the protein of SEQ ID NO: 1. Genes encoding these proteins are deposited at GenBank, wherein the GenBank accession number of a gene encoding the protein of SEQ ID NO: 5 is AL136866 (gene name; HSM801834), the GenBank accession number of a gene encoding the protein of SEQ ID NO: 6 is AK024285 (gene name; AK024285), the GenBank accession number of a gene encoding the protein of SEQ ID NO: 7 is AF167569 (gene name; AF167569), the GenBank accession number of a gene encoding the protein of SEQ ID NO: 8 is AF141870 (gene name; AF141870), the GenBank accession number of a gene encoding the protein of SEQ ID NO: 9 is AJ271745 (gene name; HSA271745), the GenBank accession number of a gene encoding the protein of SEQ ID NO: 10 is AJ271746 (gene name; HSA271746), the GenBank accession number of a gene encoding the protein of SEQ ID NO: 11 is AF167570 (gene name; AF167570), the GenBank accession number of a gene encoding the protein of SEQ ID NO: 12 is AJ271744 (gene name; HSA271744), the GenBank accession number of a gene encoding the protein of SEQ ID NO: 13 is U10324 (gene name; HSU10324), the GenBank accession number of a gene encoding the protein of SEQ ID NO: 14 is X98265 (gene name; HSMPP4II), the GenBank accession number of a gene encoding the protein of SEQ ID NO: 15 is AF147209 (gene name; AF147209), and the GenBank accession number of a gene encoding the protein of SEQ ID NO: 16 is X98264 or X98265 (gene name; HSMPP4I).

The protein having amino acid sequence of SEQ ID NO: 2 exists in a living body and comprises 366 amino acids and is referred to as human TAR RNA binding protein 2 (TRBP2). A gene encoding the protein is deposited at GenBank under accession number U08998 (gene name: HSU08998). This protein is also known to have motifs such as a motif of ribonuclease III family, dsRNA binding motif, and the like. Since the motifs of nuclease and kinase exist, the protein is considered to induce apoptosis in cells when activated.

The proteins of SEQ ID NO: 17-24 are all known proteins, which comprise amino acid sequences having high sequence identity to the amino acid sequence of dsRNA binding motif portion of the protein of SEQ ID NO: 2, and are considered to have activity equivalent to that of the protein of SEQ ID NO: 2. Genes encoding these proteins are deposited at GenBank, wherein the GenBank accession number of a gene encoding the protein of SEQ ID NO: 17 is M60801 (gene name; HUMTRBP), the GenBank accession number of a gene encoding the protein of SEQ ID NO: 18 is AF083033 (gene name; AF083033), the GenBank accession number of a gene encoding the protein of SEQ ID NO: 19 is X99227 (gene name; HSHRED1A), the GenBank accession number of a gene encoding the protein of SEQ ID NO: 20 is U18121 (gene name; HSU18121), the GenBank accession number of a gene encoding the protein of SEQ ID NO: 21 is AJ271745 (gene name; HSA271745), the GenBank accession number of a gene encoding the protein of SEQ ID NO: 22 is AF141870 (gene name; AF141870), the GenBank accession number of a gene encoding the protein of SEQ ID NO: 23 is AJ271747 (gene name; HSA271747), the GenBank accession number of a gene encoding the protein defined by SEQ ID NO: 24 is AF202445 (gene name; AF202445),

The protein having amino acid sequence of SEQ ID NO: 3 exists in a living body, comprises 803 amino acids, and is referred to as human TRA1. A gene encoding the protein is deposited at GenBank under accession number X15187 (gene name: HSTRA1). This protein is presumed to participate in the transmission of a signal for cell growth or cell death in association with other protein(s) (Saishin Igaku, vol. 56, p. 21, 2001). Examples of the other proteins include IRE1 β having nuclease motif (*e.g.*, Nature Cell Biology, vol. 3, p. 158, 2001). It is considered that, when the protein of SEQ ID NO: 3 is activated, the activity of IRE1 β is controlled and apoptosis in a cell is induced. A partial sequence of a DNA encoding IRE1 β is deposited at GenBank under accession number AA088547 (protein name: IRE1 β) (SEQ ID NO: 31).

The protein having amino acid sequence of SEQ ID NO: 4 exists in a living body, comprises 181 amino acids and is also referred to as ISG20. A gene encoding the protein is deposited at GenBank under accession number X89773 (gene name: HSISG20GN). This protein is reported to have the both of ribonuclease activity (RNase) and deoxyribonuclease activity (DNase) (e.g., Biochemistry, 40(24), p.7174, 2001). The present inventors have found that, when a complex comprising polyinosinic - polycytidylic acid double stranded RNA and cationic liposomes is added to a certain cancer cell, mRNA for said protein is expressed, which cationic liposomes are formed from 2-0-(2-diethylaminoethyl)carbamoyl-1,3-O-dioleoyl glycerol (hereinafter, referred to as "cationic glycerol") and a phospholipid as essential components, as shown in Experimental Example 2 below.

The proteins of SEQ ID NO: 25-30 are all known proteins, which comprise amino acid sequences having high sequence identity to the amino acid sequence of nuclease motif of the protein of SEQ ID NO: 4. Genes encoding these proteins are deposited at GenBank, wherein the GenBank accession number of a gene encoding the protein of SEQ ID NO: 25 is AK025493 (gene name; AK025493), the GenBank accession number of a gene encoding the protein of SEQ ID NO: 26 is AF273304 (gene name; AF273304), the GenBank accession number of a gene encoding the protein of SEQ ID NO: 27 is AF295774 (gene name; AF295774), the GenBank accession number of a gene encoding the protein of SEQ ID NO: 28 is AL136894 (gene name; HSM801862), the GenBank accession number of a gene encoding the protein of SEQ ID NO: 29 is AK022733 (gene name; AK022733), the GenBank accession number of a gene encoding the protein of SEQ ID NO: 30 is AK022546 (gene name; AK022546).

### II. Nucleotide or Nucleoside, or Pharmaceutical Composition Containing the Same

The nucleotides or nucleosides usable in the present method are not particularly restricted as long as they can exert antitumor or antivirus activity when entered into a cell. There are mono-, oligo- and poly-nucleotides, and the present invention encompasses all of them without any particular limitations.

Examples of the nucleotides or nucleoside are shown below.
(1) Homopolymer·homopolymer Double-stranded RNA
   Polyinosinic acid·polycytidylic acid,
   Polyinosinic acid·poly(5-bromocytidylic acid),
   Polyinosinic acid·poly(2-thiocytidylic acid),
   Poly(7-deazainosinic acid) ·polycytidylic acid,
   Poly(7-deazainosinic acid) ·poly(5-bromocytidylic acid),
   Poly(2'-azidoinosinic acid) ·polycytidylic acid,
   Polyinosinic acid·poly(cytidine-5'-thiophosphoric acid), and
   Polyinosinic acid·poly(1-vinylcytidylic acid).
(2) Homopolymer·copolymer Double-stranded RNA
   Polyinosinic acid·poly(cytidylic acid, uridylic acid), and
   Polyinosinic acid·poly(cytidylic acid, 4-thiouridylic acid).
(3) Copolymer Single-stranded RNA
   Poly(adenylic acid, uridylic acid).
(4) Others
   5-Fluorouracil, and
   Cytosine arabinoside.

Most of single-stranded or double-stranded RNAs among these substances cannot enter into cells directly since they are a polymer and need to be converted into a complex with, for example, a carrier for transferring/introducing a drug into cells. A polynucleotide which formed a complex with a particular drug carrier can enter into a cell. Such a carrier is available in the art or can be prepared according to a method known to one of ordinary skill in the art.

Examples of carriers useful for transferring/introducing a substance into a cell include cationic liposome carriers. Specific examples of cationic liposome carriers include a cationic liposome carrier comprising as basic essential components cationic glycerol and a phospholipid (e.g., phosphatidyl choline, phosphatidyl ethanolamine, phosphatidyl inositol, phosphatidyl serine, lecithin) in the ratio of 1:3 to 2:1 (cationic glycerol : phospholipid) by weight; Lipofectin® (a cationic liposome carrier comprising as basic essential components N-[1-(2,3-dioleyloxy)propyl]-n,n,n-trimethylammonium chloride (DOTMA) and dioleoyl phosphatidylethanolamine (DOPE) in the ratio of 1:1 by weight), Lipofectamine® (a cationic liposome carrier comprising as basic essential components 2, 3-dioleyloxy-N-[2-(sperminecarboxamido) ethyl]-N,N-dimethyl-1-propanaminium trifluoroacetate (DOSPA) and dioleoyl phosphatidylethanolamine (DOPE) in the ratio of 3:1 by weight), Cellfectin® (a cationic liposome carrier comprising as basic essential components N, N^{I}, N^{II}, N^{III}-tetramethyl-N, N^{I}, N^{II}, N^{III}-tetrapalmitylspermine (TMTPS) and dioleoyl phosphatidylethanolamine (DOPE) in the ratio of 1:1.5 by weight), DMRIE-C® (a cationic liposome carrier comprising as basic essential components 1,2-dimyristyloxypropyl-3-dimethyl-hydroxyethyl ammonium bromide (DMRIE) and cholesterol in the molar ratio of 1:1). Also included are cationic liposome carriers comprising as basic essential components any one of various glycerol derivatives described in WO94/19314 and a phospholipid (ibid). Among them, a cationic liposome carrier comprising as basic essential components a cationic glycerol and a phospholipid (e.g., lecithin) in the ratio of 1:2 to 1:1 (cationic glycerol : phospholipid) by weight is preferred.

The cationic liposome carrier consists of a lipid bilayer similarly to ordinary liposomes and is an endoplasmic reticulum that is positively charged in aqueous solution. Accordingly, the cationic liposome carrier easily forms a complex with a compound that is negatively charged in aqueous solution such as a nucleic acid.

When the nucleotide used in the present invention is a polynucleotide such as polyinosinic - polycytidylic acid, it can have various chain lengths or molecular weights, and the present invention is by no means restricted to a polynucleotide of any particular chain length or molecular weight. As to a copolymer polynucleotide such as poly(cytidylic acid, uridylic acid), it may contain two nucleotides in various constitutions or ratios, and the present invention is by no means restricted to a polynucleotide of any particular constitution or ratio. However, in the preferred constitution for the polyinosinic acid · poly(cytidylic acid, uridylic acid), one uridylic acid appears as compared with about 12 cytidylic acids (poly C₁₂U). With regard to a copolymer single-stranded RNA such as poly(adenylic acid, uridylic acid), it is preferably a constitution where two nucleotides appear alternately.

The present method is applicable to examination of efficacy of treatment with any types of nucleotide or nucleoside; however, it is suitable for examining or evaluating efficacy of treatment with polyinosinic - polycytidylic acid, and is more suitable for examining efficacy of treatment with polyinosinic - polycytidylic acid of average chain length between 100 bp and 500 bp (base pairs). As mentioned above, polyinosinic - polycytidylic acid cannot enter into a cell directly and should be administered as a complex with a cationic liposome carrier which, for example, comprises as basic essential components a cationic glycerol and a phospholipid (e.g., lecithin) in the ratio of 1:2 to 1:1 (cationic glycerol : phospholipid) by weight so that it can enter into a cell.

The present method can be conducted both *in vivo* and *in vitro.* In the former case, examples of tissues of a subject to which a nucleotide or a nucleoside is administered include vein, hepatic artery, portal vein and tumor tissues. In the latter case, examples of tissues to be taken from a subject include a carcinoma tissue and a tissue infected with virus. Specific examples of carcinoma tissues include hepatic carcinoma, lung cancer, carcinoma of colon and rectum, gastric cancer, bladder carcinoma, ovarian carcinoma, uterine cancer, breast cancer, prostatic cancer, brain tumor, kidney cancer, pancreas cancer, skin cancer and myosarcoma. However, the present invention is not limited to the above tissues, and one can select tissues appropriately depending on the purpose.

According to the present method, a nucleotide or a nucleoside is generally administered as a solution. The nucleotide or nucleoside can be in any form before being converted into a solution, including liquid preparation, powder preparation and lyophilized preparation. The present invention is not restricted to a particular form.

In the present examination method, any of known pharmaceutical compositions containing a nucleotide or a nucleoside that can enter into cells can be used as it is, which composition comprises, for example, a complex between a double stranded RNA such as polyinosinic - polycytidylic acid and a cationic liposome carrier. Such a pharmaceutical composition can be prepared in a conventional manner or are commercially available.

The dose of a nucleotide or a nucleoside should vary depending on the kinds of nucleotide or nucleoside to be used, the disease, conditions of the patient, or the like, and is appropriately determined.

### III. Determination of Expression Amount of Protein

In the present examination method, the determination of the expression amount of a protein according to the present invention can be conducted by a known method wherein (1) the target protein is measured directly, or (2) the expression of mRNA encoding the target protein is measured, and the like. Specific examples of the former include ELISA method that uses an appropriate antibody and specific examples of the latter include RT-PCR method.

When carrying out the present method, the expression amount of a protein according to the present invention is measured in vivo or in vitro, before and after the administration of a nucleotide or a nucleoside into a tissue of a subject or into a tissue obtained from the subject. The increase in the amount of expression of the protein as a result of administration of the nucleotide or the nucleoside leads to the evaluation that a pharmaceutical composition containing the nucleotide or the nucleoside possibly has therapeutic effect in said patient.

Among the proteins according to the present invention, preferred protein of which expression amount is measured varies depending on the kind of nucleotide or nucleoside to be used, the kind of disease (*e*.*g*., tumor, infected virus, *etc.*), conditions of the patient, and the like. To examine therapeutic efficacy of a pharmaceutical composition containing as a double-stranded RNA polyinosinic - polycytidylic acid and cationic liposome carrier, it would be useful to measure the protein having amino acid sequence of SEQ ID NO: 4. When the expression amount of the protein of SEQ ID NO: 4 increases after the administration of said pharmaceutical composition, said composition can be estimated to have therapeutic effect in said patient.

The criterion of judgment regarding therapeutic efficacy should vary depending on the condition of a patient, kind of nucleotide or nucleoside to be used, the kind of disease (e.g., tumor, infected virus, etc.), a protein to be measured, method of measurement, and the like. However, if the expression amount of a protein measured after the administration increased 1.5 or more times, preferably, 2.0 or more times as compared to what measured before the administration, it can be estimated to be therapeutically effective.

### IV. Determination of Activity of Protein

In the present examination method, the determination of the activity of a protein according to the present invention can be conducted by a known method. Examples of such activity include nuclease (DNase, RNase) activity, kinase activity and protein-binding activity. Specific examples of the method for determining the activity involve the determination of nuclease (DNase, RNase) activity or kinase activity as hereinafter described.

### (1) Determination of DNase Activity

### Substrate:

Cell fraction, DNA or DNA plasmid, which are radio-labeled with a radioisotope such as radioactive phosphorous

### Reaction solution:

50mM MES-NaOH(pH5.6) or 10 mM Tris-HCl (pH7.8), 3 mM CaCl₂, 3 mM MgCl₂, 0.1 mM PMSF, 1 mM mercaptoethanol

To the substrate and the reaction solution is added a test protein and the mixture is incubated at 37 °C for 10 to 60 minutes. The reaction mixture is centrifuged at 20,000 x g for 20 seconds and the supernatant is removed. The precipitates are treated with RNase and proteinase K, and electrophoresed on agarose. The DNase activity can be determined from the amount of decomposed radio-labeled DNA as an indicator.

### (2) Determination of RNase Activity

### Substrate:

Ribosomal RNA radio-labeled with a radioisotope such as radioactive phosphorous

### Reaction solution:

10 mM Tris-HCl(pH7.4), 300 mM NaCl, 5 mM EDTA

To the substrate and the reaction solution is added a test protein and the mixture is incubated at 37 °C for 10 to 60 minutes. The radio-labeled ribosomal RNA is extracted by guanidium thiocyanate method and electrophoresed on agarose. The RNase activity can be determined from the amount of fragmented radio-labeled RNA as an indicator.

### (3) Determination of Kinase Activity

### Substrate:Peptide

### Reaction solution:

20-50 mM Tris-HCl (pH7.4), 5-10 mM MgCl₂, 250-500 µ M
[ γ -³²P]ATP, 15 mM mercaptoethanol, 0.1% Tween80

To the substrate and the reaction solution is added a test protein and the mixture is incubated at 37 °C for 10 to 60 minutes. The substrate peptides are extracted and the kinase activity can be determined from the amount of radio-labeled peptides as an indicator.

When carrying out the present method, the nuclease activity or the like is measured *in vivo* or *in vitro,* before and after the administration of a nucleotide or a nucleoside into a tissue of a subject or into a tissue obtained from the subject. The increase in the activity as a result of administration of the nucleotide or the nucleoside leads to the evaluation that a pharmaceutical composition containing the said nucleotide or the nucleoside possibly has therapeutic effect in said patient.

Among the proteins according to the present invention, preferred protein of which expression amount should be measured varies depending on the kind of nucleotide or nucleoside to be used, the kind of disease (e.g., tumor, infected virus, etc.), conditions of the patient, and the like. To examine therapeutic efficacy of a pharmaceutical composition containing as a double-stranded RNA polyinosinic - polycytidylic acid and cationic liposome carrier, it would be useful to measure a protein from those having the amino acid sequences of SEQ ID NO: 1-4, preferably SEQ ID NO: 1, 2 and 4. When the expression amount of a protein having an amino acid sequence selected from SEQ ID NO: 1-4 increases after the administration of said pharmaceutical composition, said composition can be estimated as being possibly therapeutically effective in said patient.

The criterion of judgment regarding therapeutic efficacy varies depending on the kind of nucleotide or nucleoside to be used, condition of a patient, the kind of disease (e.g., tumor, infected virus, etc.), a protein to be measured, method of measurement, and the like. However, if the activity of a protein measured increased 1.5 or more times, preferably, 2.0 or more times as compared to what measured before the administration, it can be estimated as being therapeutically effective.

### V. Determination of Binding Ability to Proteins

Examples of method for examining the binding ability of a test substance (medical substance) to a protein according to the present invention include West western method with an appropriate antibody, immunoprecipitation method, and a process by means of BIAcore® (Pharmacia Biotech) which is an automated analytic device based on surface plasmon resonance (SPR), an optical phenomenon, as measuring principle. If a test substance proved to bind to a protein(s) according to the present invention, said test substance can be a promising antitumor or antivirus agent.

A substance known to have antitumor or antivirus activity can also be examined for the ability to bind to a protein of the present invention according to a similar method as the above. If the substance proved to bind to the protein, said substance can be expected to act in vivo through at least a similar reaction mechanism as polyinosinic - polycytidylic acid.

The experimental examples below provides the detection or identification of a protein(s) according to the present invention, and influence of a nucleic acid on the expression of mRNA for the protein. In the following experimental examples, a double-stranded RNA, polyinosinic acid·polycytidylic acid (polyinosinic - polycytidylic acid, hereinafter, referred to as "poly(I)·poly(C)") was used as a nucleic acid-containing test substance (drug). Poly(I) · poly(C) is known to be efficacious against certain cancer cells (e.g., HeLaS3, A431) but inefficacious against others (e.g., HeLa, SW620) (Cancer Research, 59, pp. 4325-4333 (1999)).

### Experimental Example 1

### Detection of Binding Ability to Proteins of SEQ ID NO: 1-3

(1) Each protein was synthesized by plating phages containing cDNA on a petri dish using human HeLa 5'-STRETCH PLUS cDNA library.
(2) The synthesized protein was transferred to nitrocellulose membrane.
(3) To the nitrocellulose membrane was added the following test solution containing poly(I) · poly(C) (chain length 100-500bp) radio-labeled with [γ-³²P]ATP, and subjected to hybridization at 42°C, for 5-6 hours.

### <Test solution>

10 mM HEPES, pH7.5
50 mM NaCl
1 mM Dithiothreitol
1 mM EDTA
5% Glycerol
1 ng/ml - 1µg/ml [³²P]-poly(I) · poly(C)

(4) The nitrocellulose membrane was washed several times with above-mentioned solution which does not contain[³²P]-poly(I) · poly(C) and exposed to imaging plate to screen proteins bound to poly(I) · poly(C). The nucleotide sequence of cDNA corresponding to the screened protein was determined with a sequencer to identify the screened protein.

As a result, proteins of SEQ ID NO: 1-3 were revealed to that bound to poly(I) ·poly(C).

### Experimental Example 2

### Detection of Expression of mRNA for Protein of SEQ ID NO: 4 (ISG20)

(1) A test complex comprising poly(I) · poly(C) was added to each of cancer cells, Hela, HeLaS3, A431 and SW620 cells so that the concentration of poly(I) · poly(C) becomes from 10 to 100 ng/ml.
(2) Following the addition of the test complex, total RNA was extracted from HeLa and HeLaS3 cells at 2 and 4 hours, and from A431 and SW620 cells at 0.5, 1, 2 and 4 hours by guanidium thiocyanate method.
(3) The extracted RNA was subjected to the Reverse Transcriptase Chain Reaction (RT-PCR) and the reaction solution was electrophoresed on agarose gel. The bands indicated by white arrow in Figs. 2 and 3 show ISG20 mRNA.

### <Test Complex>

| | Weight ratio |
|---|---|
| Poly (I) • poly(C) (chain length 100∼500bp) | 1 |
| Cationic glycerol | 6 |
| Yolk lecithin | 10 |

As is clear from Figs. 2 and 3, the expression amount of ISG20 mRNA increased with time in cancer cells (HeLaS3, A431) on which the complex comprising poly(I) • poly(C) has effect. On the other hand, ISG20 mRNA was not expressed in cancer cells (HeLa, SW620) on which the complex comprising poly(I) · poly(C) does not have effect. This result shows that it is possible to evaluate whether a test drug is effective as a therapeutic agent in a specific patient on the basis of the presence or absence of expression of ISG20.

### Industrial Applicability

According to the method of the present invention, the efficacy of an antitumor or antivirus agent comprising a nucleic acid in individual patients can be evaluated beforehand, and hence it becomes possible to treat a patient more effectively through tailor-made therapy or the like. Furthermore, according to the present method, a substance potentially having antitumor or antivirus activity can be screened.

## Claims

1. A method of examining efficacy of treatment with a nucleotide or a nucleoside in a given subject comprising:
(a) measuring the amount of expression or activity of at least one protein among those each having amino acid sequences of SEQ ID NO: 1-4 in a blood or tissue sample previously taken from the subject;
(b) administering the nucleotide or the nucleoside into a tissue of the subject or a tissue taken from the subject followed by measuring the amount of expression or activity of the same protein as that measured in (a) in blood or treated tissue after 3-48 hours from administration; and
(c) comparing the measurement obtained in step (a) with that obtained in step (b) and evaluating the change in the amount of expression or the activity of the protein.

2. A method of examining efficacy of treatment with a nucleotide or a nucleoside in a given subject comprising:
(a) measuring the amount of expression or activity of at least one protein among those each having amino acid sequences of SEQ ID NO: 5-30 in a blood or tissue sample previously taken from the subject;
(b) administering the nucleotide or the nucleoside into a tissue of the subject or a tissue taken from the subject followed by measuring the amount of expression or activity of the same protein as that measured in (a) in blood or treated tissue after 3-48 hours from administration; and
(c) comparing the measurement obtained in step (a) with that obtained in step (b) and evaluating the change in the amount of expression or the activity of the protein.

3. The method of claim 1 or 2, wherein the nucleotide or nucleoside exerts antitumor or antivirus activity at least when entered into a cell and is used in the form of a complex with a carrier effective to transfer the same into a cell.

4. The method of claim 3, wherein the carrier is cationic liposome carrier.

5. The method of claim 4, wherein the cationic liposome carrier is a cationic liposome carrier comprising as basic essential components 2-O-(2-diethylaminoethyl)carbamoyl-1,3-O-dioleoyl glycerol and a phospholipid in the ratio of 1:3 to 2:1 (the glycerol derivative : the phospholipid) by weight; Lipofectin® (a cationic liposome carrier comprising as basic essential components N-[1-(2,3-dioleyloxy)propyl]-n,n,n-trimethylammonium chloride (DOTMA) and dioleoyl phosphatidylethanolamine (DOPE) in the ratio of 1:1 by weight), Lipofectamine® (a cationic liposome carrier comprising as basic essential components 2, 3-dioleyloxy-N-[2-(sperminecarboxamido) ethyl]-N,N-dimethyl-1-propanammonium trifluoroacetate (DOSPA) and dioleoyl phosphatidylethanolamine (DOPE) in the ratio of 3:1 by weight), Cellfectin® (a cationic liposome carrier comprising as basic essential components N, N^{I}, N^{II}, N^{III}-tetramethyl-N, N^{I}, N^{II}, N^{III}-tetrapalmitylspermine (TMTPS) and dioleoyl phosphatidylethanolamine (DOPE) in the ratio of 1:1.5 by weight), or DMRIE-C® (a cationic liposome carrier comprising as basic essential components 1,2-dimyristyloxypropyl-3-dimethyl-hydroxyethyl ammonium bromide (DMRIE) and cholesterol in the molar ratio of 1:1).

6. The method of claim 1 or 2, wherein the nucleotide or the nucleoside is a homopolymer · homopolymer double-stranded RNA, homopolymer · copolymer double-stranded RNA, copolymer single-stranded RNA, 5-Fluorouracil, or cytosine arabinoside.

7. The method of claim 6, wherein the homopolymer · homopolymer double-stranded RNA is selected from the group consisting of polyinosinic acid · polycytidylic acid, polyinosinic acid · poly(5-bromocytidylic acid), polyinosinic acid · poly(2-thiocytidylic acid), poly(7-deazainosinic acid) · polycytidylic acid, poly(7-deazainosinic acid) · poly(5-bromocytidylic acid), poly(2'-azidoinosinic acid) · polycytidylic acid, polyinosinic acid · poly(cytidine-5'-thiophosphoric acid), and polyinosinic acid·poly(1-vinylcytidylic acid).

8. The method of claim 6, wherein the homopolymer · copolymer double-stranded RNA is polyinosinic acid · poly(cytidylic acid, uridylic acid) or polyinosinic acid · poly(cytidylic acid, 4-thiouridylic acid).

9. The method of claim 6, wherein the copolymer single-stranded RNA is poly(adenylic acid, uridylic acid).

10. A method of examining efficacy of treatment with a complex of polyinosinic acid·polycytidylic acid of average chain length between 100 bp and 500 bp with a cationic liposome carrier which comprises as basic essential components 2-O-(2-diethylaminoethyl)carbamoyl-1,3-O-dioleoyl glycerol and a phospholipid in the ratio of 1:3 to 2:1 (the glycerol derivative : the phospholipid) by weight in a given subject comprising:
(a) measuring the amount of expression or activity of at least one protein among those each having amino acid sequences of SEQ ID NO: 1-4 in a blood or tissue sample previously taken from the subject;
(b) administering the nucleotide or the nucleoside into a tissue of the subject or a tissue taken from the subject followed by measuring the amount of expression or activity of the same protein as that measured in (a) in blood or treated tissue after 3-48 hours from administration; and
(c) comparing the measurement obtained in step (a) with that obtained in step (b) and evaluating the change in the amount of expression or the activity of the protein.

11. A pharmaceutical composition for examining efficacy of treatment with a nucleotide or a nucleoside in a given subject comprising:
(a) measuring the amount of expression or activity of at least one protein among those each having amino acid sequences of SEQ ID NO: 1-4 in a blood or tissue sample previously taken from the subject;
(b) administering the nucleotide or the nucleoside into a tissue of the subject or a tissue taken from the subject followed by measuring the amount of expression or activity of the same protein as that measured in (a) in blood or treated tissue after 3-48 hours from administration; and
(c) comparing the measurement obtained in step (a) with that obtained in step (b) and evaluating the change in the amount of expression or the activity of the protein.

12. A pharmaceutical composition for examining efficacy of treatment with a nucleotide or a nucleoside in a given subject comprising:
(a) measuring the amount of expression or activity of at least one protein among those each having amino acid sequences of SEQ ID NO: 5-30 in a blood or tissue sample previously taken from the subject;
(b) administering the nucleotide or the nucleoside into a tissue of the subject or a tissue taken from the subject followed by measuring the amount of expression or activity of the same protein as that measured in (a) in blood or treated tissue after 3-48 hours from administration; and
(c) comparing the measurement obtained in step (a) with that obtained in step (b) and evaluating the change in the amount of expression or the activity of the protein.

13. A pharmaceutical composition of claim 11 or 12, wherein the nucleotide or nucleoside exerts antitumor or antivirus activity at least when entered into a cell and is used in the form of a complex with a carrier effective to transfer the same into a cell.

14. A pharmaceutical composition of claim 13, wherein the carrier is cationic liposome carrier.

15. A pharmaceutical composition of claim 14, wherein the cationic liposome carrier is a cationic liposome carrier comprising as basic essential components 2-O-(2-diethylaminoethyl)carbamoyl-1,3-O-dioleoyl glycerol and a phospholipid in the ratio of 1:3 to 2:1 (the glycerol derivative : the phospholipid) by weight; Lipofectin® (a cationic liposome carrier comprising as basic essential components N-[1-(2,3-dioleyloxy)propyl]-n,n,n-trimethylammonium chloride (DOTMA) and dioleoyl phosphatidylethanolamine (DOPE) in the ratio of 1:1 by weight), Lipofectamine® (a cationic liposome carrier comprising as basic essential components 2, 3-dioleyloxy-N-[2-(sperminecarboxamido) ethyl]-N,N-dimethyl-1-propanammonium trifluoroacetate (DOSPA) and dioleoyl phosphatidylethanolamine (DOPE) in the ratio of 3:1 by weight), Cellfectin® (a cationic liposome carrier comprising as basic essential components N, N^{I}, N^{II}, N^{III}-tetramethyl-N, N^{I}, N^{II}, N^{III}-tetrapalmitylspermine (TMTPS) and dioleoyl phosphatidylethanolamine (DOPE) in the ratio of 1:1.5 by weight), or DMRIE-C® (a cationic liposome carrier comprising as basic essential components 1,2-dimyristyloxypropyl-3-dimethyl-hydroxyethyl ammonium bromide (DMRIE) and cholesterol in the molar ratio of 1:1).

16. A pharmaceutical composition of claim 11 or 12, wherein the nucleotide or the nucleoside is a homopolymer· homopolymer double-stranded RNA, homopolymer · copolymer double-stranded RNA, copolymer single-stranded RNA, 5-Fluorouracil, or cytosine arabinoside.

17. A pharmaceutical composition of claim 16, wherein the homopolymer · homopolymer double-stranded RNA is selected from the group consisting of polyinosinic acid · polycytidylic acid, polyinosinic acid · poly(5-bromocytidylic acid), polyinosinic acid · poly(2-thiocytidylic acid), poly(7-deazainosinic acid) · polycytidylic acid, poly(7-deazainosinic acid) · poly(5-bromocytidylic acid), poly(2'-azidoinosinic acid) · polycytidylic acid, polyinosinic acid · poly(cytidine-5'-thiophosphoric acid), and polyinosinic acid · poly(1-vinylcytidylic acid).

18. A pharmaceutical composition of claim 16, wherein the homopolymer · copolymer double-stranded RNA is polyinosinic acid · poly(cytidylic acid, uridylic acid) or polyinosinic acid · poly(cytidylic acid, 4-thiouridylic acid).

19. A pharmaceutical composition of claim 16, wherein the copolymer single-stranded RNA is poly(adenylic acid, uridylic acid).

20. A pharmaceutical composition for examining efficacy of treatment with a complex of polyinosinic acid polycytidylic acid of average chain length between 100 bp and 500 bp with a cationic liposome carrier which comprises as basic essential components 2-O-(2-diethylaminoethyl)carbamoyl-1,3-O-dioleoyl glycerol and a phospholipid in the ratio of 1:3 to 2:1 (the glycerol derivative : the phospholipid) by weight in a given subject comprising:
(a) measuring the amount of expression or activity of at least one protein among those each having amino acid sequences of SEQ ID NO: 1-4 in a blood or tissue sample previously taken from the subject;
(b) administering the nucleotide or the nucleoside into a tissue of the subject or a tissue taken from the subject followed by measuring the amount of expression or activity of the same protein as that measured in (a) in blood or treated tissue after 3-48 hours from administration; and
(c) comparing the measurement obtained in step (a) with that obtained in step (b) and evaluating the change in the amount of expression or the activity of the protein.

21. A method of screening for agents potentially having antitumor or antivirus activity comprising examining a test compound for ability to bind to at least one protein among those each having amino acid sequences of SEQ ID NO: 1-4.

22. A method of screening for agents potentially having antitumor or antivirus activity comprising examining a test compound for ability to bind to at least one protein among those each having amino acid sequences of SEQ ID NO: 5-30.

23. A method of investigating the mechanism of action of an antitumor or antivirus agent *in vivo* comprising examining the antitumor or antivirus agent for ability to bind to at least one protein among those each having amino acid sequences of SEQ ID NO: 1-4.

24. A method of investigating the mechanism of action of an antitumor or antivirus agent *in vivo* comprising examining the antitumor or antivirus agent for ability to bind to at least one protein among those each having amino acid sequences of SEQ ID NO: 5-30.


SEQUIENCE LISTING

<110> NIPPON SHINYAKU CO., LTD.
<120> METHOD OF EVALUATING EFFICACY OF NUCLEIC ACID THERAPY
<130> B-345
<160> 31
<210> 1
<211> 490
<212> PRT
<213> Homo sapiens
<400> 1
Met Lys Asp Pro Pro Asp Leu Leu Asp Arg Gln Lys Cys Pro Asn Ala
1               5                   10                  15
Leu Ala Ser Leu Arg His Ala Lys Trp Phe Gln Ala Arg Ala Asn Gly
            20                  25                  30
Leu Lys Ser Cys Val Ile Val Leu Arg Ile Leu Arg Asp Leu Cys Asn
        35                  40                  45
Arg Val Pro Thr Trp Ala Pro Leu Lys Gly Trp Pro Leu Glu Leu Ile
    50                  55                  60
Cys Glu Lys Ser Ile Gly Thr Cys Asn Arg Pro Leu Gly Ala Gly Glu
65                  70                  75                  80
Ala Leu Arg Arg Val Met Glu Cys Leu Ala Ser Gly Ile Leu Leu Pro
                85                  90                  95
Gly Gly Pro Gly Leu His Asp Pro Cys Glu Arg Asp Pro Thr Asp Ala
            100                 105                 110
Leu Ser Tyr Met Thr Ile Gln Gln Lys Glu Asp Ile Thr His Ser Ala
        115                 120                 125
Gln His Ala Leu Arg Leu Ser Ala Phe Gly Gln Ile Tyr Lys Val Leu
    130                 135                 140
Glu Met Asp Pro Leu Pro Ser Ser Lys Pro Phe Gln Lys Tyr Ser Trp
145                 150                 155                 160
Ser Val Thr Asp Lys Glu Gly Ala Gly Ser Ser Ala Leu Lys Arg Pro
                165                 170                 175
Phe Glu Asp Gly Leu Gly Asp Asp Lys Asp Pro Asn Lys Lys Met Lys
            180                 185                 190
Arg Asn Leu Arg Lys Ile Leu Asp Ser Lys Ala Ile Asp Leu Met Asn
        195                 200                 205
Ala Leu Met Arg Leu Asn Gln Ile Arg Pro Gly Leu Gln Tyr Lys Leu
    210                 215                 220
Leu Ser Gln Ser Gly Pro Val His Ala Pro Val Phe Thr Met Ser Val
225                 230                 235                 240
Asp Val Asp Gly Thr Thr Tyr Glu Ala Ser Gly Pro Ser Lys Lys Thr
                245                 250                 255
Ala Lys Leu His Val Ala Val Lys Val Leu Gln Ala Met Gly Tyr Pro
            260                 265                 270

Thr Gly Phe Asp Ala Asp Ile Glu Cys Met Ser Ser Asp Glu Lys Ser
        275                 280                 285
Asp Asn Glu Ser Lys Asn Glu Thr Val Ser Ser Asn Ser Ser Asn Asn
    290                 295                 300
Thr Gly Asn Ser Thr Thr Glu Thr Ser Ser Thr Leu Glu Val Arg Thr
305                 310                 315                 320
Gln Gly Pro Ile Leu Thr Ala Ser Gly Lys Asn Pro Val Met Glu Leu
                325                 330                 335
Asn Glu Lys Arg Arg Gly Leu Lys Tyr Glu Leu Ile Ser Glu Thr Gly
            340                 345                 350
Gly Ser His Asp Lys Arg Phe Val Met Glu Val Glu Val Asp Gly Gln
        355                 360                 365
Lys Phe Arg Gly Ala Gly Pro Asn Lys Lys Val Ala Lys Ala Ser Ala
    370                 375                 380
Ala Leu Ala Ala Leu Glu Lys Leu Phe Ser Gly Pro Asn Ala Ala Asn
385                 390                 395                 400
Asn Lys Lys Lys Lys Ile Ile Pro Gln Ala Lys Gly Val Val Asn Thr
                405                 410                 415
Ala Val Ser Ala Ala Val Gln Ala Val Arg Gly Arg Gly Arg Gly Thr
            420                 425                 430
Leu Thr Arg Gly Ala Phe Val Gly Ala Thr Ala Ala Pro Gly Tyr Ile
        435                 440                 445
Ala Pro Gly Tyr Gly Thr Pro Tyr Gly Tyr Ser Thr Ala Ala Pro Ala
    450                 455                 460
Tyr Gly Leu Pro Lys Arg Met Val Leu Leu Pro Val Met Lys Phe Pro
465                 470                 475                 480
Thr Tyr Pro Val Pro His Tyr Ser Phe Phe
                485                 490

<210> 2
<211> 366
<212> PRT
<213> Homo sapiens
<400> 2
Met Ser Glu Glu Glu Gln Gly Ser Gly Thr Thr Thr Gly Cys Gly Leu
  1               5                  10                  15
Pro Ser Ile Glu Gln Met Leu Ala Ala Asn Pro Gly Lys Thr Pro Ile
             20                  25                  30
Ser Leu Leu Gln Glu Tyr Gly Thr Arg Ile Gly Lys Thr Pro Val Tyr
         35                  40                  45
Asp Leu Leu Lys Ala Glu Gly Gln Ala His Gln Pro Asn Phe Thr Phe
     50                  55                  60
Arg Val Thr Val Gly Asp Thr Ser Cys Thr Gly Gln Gly Pro Ser Lys
 65                  70                  75                  80
Lys Ala Ala Lys His Lys Ala Ala Glu Val Ala Leu Lys His Leu Lys
                 85                  90                  95

Gly Gly Ser Met Leu Glu Pro Ala Leu Glu Asp Ser Ser Ser Phe Ser
            100                 105                 110
Pro Leu Asp Ser Ser Leu Pro Glu Asp Ile Pro Val Phe Thr Ala Ala
        115                 120                 125
Ala Ala Ala Thr Pro Val Pro Ser Val Val Leu Thr Arg Ser Pro Pro
    130                 135                 140
Met Glu Leu Gln Pro Pro Val Ser Pro Gln Gln Ser Glu Cys Asn Pro
145                 150                 155                 160
Val Gly Ala Leu Gln Glu Leu Val Val Gln Lys Gly Trp Arg Leu Pro
                165                 170                 175
Glu Tyr Thr Val Thr Gln Glu Ser Gly Pro Ala His Arg Lys Glu Phe
            180                 185                 190
Thr Met Thr Cys Arg Val Glu Arg Phe Ile Glu Ile Gly Ser Gly Thr
        195                 200                 205
Ser Lys Lys Leu Ala Lys Arg Asn Ala Ala Ala Lys Met Leu Leu Arg
    210                 215                 220
Val His Thr Val Pro Leu Asp Ala Arg Asp Gly Asn Glu Val Glu Pro
225                 230                 235                 240
Asp Asp Asp His Phe Ser Ile Gly Val Gly Phe Arg Leu Asp Gly Leu
                245                 250                 255
Arg Asn Arg Gly Pro Gly Cys Thr Trp Asp Ser Leu Arg Asn Ser Val
            260                 265                 270
Gly Glu Lys Ile Leu Ser Leu Arg Ser Cys Ser Leu Gly Ser Leu Gly
        275                 280                 285
Ala Leu Gly Pro Ala Cys Cys Arg Val Leu Ser Glu Leu Ser Glu Glu
    290                 295                 300
Gln Ala Phe His Val Ser Tyr Leu Asp Ile Glu Glu Leu Ser Leu Ser
305                 310                 315                 320
Gly Leu Cys Gln Cys Leu Val Glu Leu Ser Thr Gln Pro Ala Thr Val
                325                 330                 335
Cys His Gly Ser Ala Thr Thr Arg Glu Ala Ala Arg Gly Glu Ala Ala
            340                 345                 350
Arg Arg Ala Leu Gln Tyr Leu Lys Ile Met Ala Gly Ser Lys
        355                 360                 365

<210> 3
<211> 803
<212> PRT
<213> Homo sapiens
<400> 3
Met Arg Ala Leu Trp Val Leu Gly Leu Cys Cys Val Leu Leu Thr Phe
1               5                   10                  15
Gly Ser Val Arg Ala Asp Asp Glu Val Asp Val Asp Gly Thr Val Glu
            20                  25                  30
Glu Asp Leu Gly Lys Ser Arg Glu Gly Ser Arg Thr Asp Asp Glu Val
        35                  40                  45

Val Gln Arg Glu Glu Glu Ala Ile Gln Leu Asp Gly Leu Asn Ala Ser
    50                  55                  60
Gln Ile Arg Glu Leu Arg Glu Lys Ser Glu Lys Phe Ala Phe Gln Ala
65                  70                  75                  80
Glu Val Asn Arg Met Met Lys Leu Ile Ile Asn Ser Leu Tyr Lys Asn
                85                  90                  95
Lys Glu Ile Phe Leu Arg Glu Leu Ile Ser Asn Ala Ser Asp Ala Leu
            100                 105                 110
Asp Lys Ile Arg Leu Ile Ser Leu Thr Asp Glu Asn Ala Leu Ser Gly
        115                 120                 125
Asn Glu Glu Leu Thr Val Lys Ile Lys Cys Asp Lys Glu Lys Asn Leu
    130                 135                 140
Leu His Val Thr Asp Thr Gly Val Gly Met Thr Arg Glu Glu Leu Val
145                 150                 155                 160
Lys Asn Leu Gly Thr Ile Ala Lys Ser Gly Thr Ser Glu Phe Leu Asn
                165                 170                 175
Lys Met Thr Glu Ala Gln Glu Asp Gly Gln Ser Thr Ser Glu Leu Ile
            180                 185                 190
Gly Gln Phe Gly Val Gly Phe Tyr Ser Ala Phe Leu Val Ala Asp Lys
        195                 200                 205
Val Ile Val Thr Ser Lys His Asn Asn Asp Thr Gln His Ile Trp Glu
    210                 215                 220
Ser Asp Ser Asn Glu Phe Ser Val Ile Ala Asp Pro Arg Gly Asn Thr
225                 230                 235                 240
Leu Gly Arg Gly Thr Thr Ile Thr Leu Val Leu Lys Glu Glu Ala Ser
                245                 250                 255
Asp Tyr Leu Glu Leu Asp Thr Ile Lys Asn Leu Val Lys Lys Tyr Ser
            260                 265                 270
Gln Phe Ile Asn Phe Pro Ile Tyr Val Trp Ser Ser Lys Thr Glu Thr
        275                 280                 285
Val Glu Glu Pro Met Glu Glu Glu Glu Ala Ala Lys Glu Glu Lys Glu
    290                 295                 300
Glu Ser Asp Asp Glu Ala Ala Val Glu Glu Glu Glu Glu Glu Lys Lys
305                 310                 315                 320
Pro Lys Thr Lys Lys Val Glu Lys Thr Val Trp Asp Trp Glu Leu Met
                325                 330                 335
Asn Asp Ile Lys Pro Ile Trp Gln Arg Pro Ser Lys Glu Val Glu Glu
            340                 345                 350
Asp Glu Tyr Lys Ala Phe Tyr Lys Ser Phe Ser Lys Glu Ser Asp Asp
        355                 360                 365
Pro Met Ala Tyr Ile His Phe Thr Ala Glu Gly Glu Val Thr Phe Lys
    370                 375                 380
Ser Ile Leu Phe Val Pro Thr Ser Ala Pro Arg Gly Leu Phe Asp Glu
385                 390                 395                 400
Tyr Gly Ser Lys Lys Ser Asp Tyr Ile Lys Leu Tyr Val Arg Arg Val
                405                 410                 415

Phe Ile Thr Asp Asp Phe His Asp Met Met Pro Lys Tyr Leu Asn Phe
            420                 425                 430
Val Lys Gly Val Val Asp Ser Asp Asp Leu Pro Leu Asn Val Ser Arg
        435                 440                 445
Glu Thr Leu Gln Gln His Lys Leu Leu Lys Val Ile Arg Lys Lys Leu
    450                 455                 460
Val Arg Lys Thr Leu Asp Met Ile Lys Lys Ile Ala Asp Asp Lys Tyr
465                 470                 475                 480
Asn Asp Thr Phe Trp Lys Glu Phe Gly Thr Asn Ile Lys Leu Gly Val
                485                 490                 495
Ile Glu Asp His Ser Asn Arg Thr Arg Leu Ala Lys Leu Leu Arg Phe
            500                 505                 510
Gln Ser Ser His His Pro Thr Asp Ile Thr Ser Leu Asp Gln Tyr Val
        515                 520                 525
Glu Arg Met Lys Glu Lys Gln Asp Lys Ile Tyr Phe Met Ala Gly Ser
    530                 535                 540
Ser Arg Lys Glu Ala Glu Ser Ser Pro Phe Val Glu Arg Leu Leu Lys
545                 550                 555                 560
Lys Gly Tyr Glu Val Ile Tyr Leu Thr Glu Pro Val Asp Glu Tyr Cys
                565                 570                 575
Ile Gln Ala Leu Pro Glu Phe Asp Gly Lys Arg Phe Gln Asn Val Ala
            580                 585                 590
Lys Glu Gly Val Lys Phe Asp Glu Ser Glu Lys Thr Lys Glu Ser Arg
        595                 600                 605
Glu Ala Val Glu Lys Glu Phe Glu Pro Leu Leu Asn Trp Met Lys Asp
    610                 615                 620
Lys Ala Leu Lys Asp Lys Ile Glu Lys Ala Val Val Ser Gln Arg Leu
625                 630                 635                 640
Thr Glu Ser Pro Cys Ala Leu Val Ala Ser Gln Tyr Gly Trp Ser Gly
                645                 650                 655
Asn Met Glu Arg Ile Met Lys Ala Gln Ala Tyr Gln Thr Gly Lys Asp
            660                 665                 670
Ile Ser Thr Asn Tyr Tyr Ala Ser Gln Lys Lys Thr Phe Glu Ile Asn
        675                 680                 685
Pro Arg His Pro Leu Ile Arg Asp Met Leu Arg Arg Ile Lys Glu Asp
    690                 695                 700
Glu Asp Asp Lys Thr Val Leu Asp Leu Ala Val Val Leu Phe Glu Thr
705                 710                 715                 720
Ala Thr Leu Arg Ser Gly Tyr Leu Leu Pro Asp Thr Lys Ala Tyr Gly
                725                 730                 735
Asp Arg Ile Glu Arg Met Leu Arg Leu Ser Leu Asn Ile Asp Pro Asp
            740                 745                 750
Ala Lys Val Glu Glu Glu Pro Glu Glu Glu Pro Glu Glu Thr Ala Glu
        755                 760                 765
Asp Thr Thr Glu Asp Thr Glu Gln Asp Glu Asp Glu Glu Met Asp Val
    770                 775                 780

Gly Thr Asp Glu Glu Glu Glu Thr Ala Lys Glu Ser Thr Ala Glu Lys
785                 790                 795                 800
Asp Glu Leu

<210> 4
<211> 181
<212> PRT
<213> Homo sapiens
<400> 4
Met Ala Gly Ser Arg Glu Val Val Ala Met Asp Cys Glu Met Val Gly
1               5                   10                  15
Leu Gly Pro His Arg Glu Ser Gly Leu Ala Arg Cys Ser Leu Val Asn
            20                  25                  30
Val His Gly Ala Val Leu Tyr Asp Lys Phe Ile Arg Pro Glu Gly Glu
        35                  40                  45
Ile Thr Asp Tyr Arg Thr Arg Val Ser Gly Val Thr Pro Gln His Met
    50                  55                  60
Val Gly Ala Thr Pro Phe Ala Val Ala Arg Leu Glu Ile Leu Gln Leu
65                  70                  75                  80
Leu Lys Gly Lys Leu Val Val Gly His Asp Leu Lys His Asp Phe Gln
                85                  90                  95
Ala Leu Lys Glu Asp Met Ser Gly Tyr Thr Ile Tyr Asp Thr Ser Thr
            100                 105                 110
Asp Arg Leu Leu Trp Arg Glu Ala Lys Leu Asp His Cys Arg Arg Val
        115                 120                 125
Ser Leu Arg Val Leu Ser Glu Arg Leu Leu His Lys Ser Ile Gln Asn
    130                 135                 140
Ser Leu Leu Gly His Ser Ser Val Glu Asp Ala Arg Ala Thr Met Glu
145                 150                 155                 160
Leu Tyr Gln Ile Ser Gln Arg Ile Arg Ala Arg Arg Gly Leu Pro Arg
                165                 170                 175
Leu Ala Val Ser Asp
            180

<210> 5
<211> 658
<212> PRT
<213> Homo sapiens
<400> 5
Met Val Lys His Ser Thr Ile Tyr Pro Ser Pro Glu Glu Leu Glu Ala
1               5                   10                  15
Val Gln Asn Met Val Ser Thr Val Glu Cys Ala Leu Lys His Val Ser
            20                  25                  30
Asp Trp Leu Asp Glu Thr Asn Lys Gly Thr Lys Thr Glu Gly Glu Thr
        35                  40                  45

Glu Val Lys Lys Asp Glu Ala Gly Glu Asn Tyr Ser Lys Asp Gln Gly
    50                  55                  60
Gly Arg Thr Leu Cys Gly Val Met Arg Ile Gly Leu Val Ala Lys Gly
65                  70                  75                  80
Leu Leu Ile Lys Asp Asp Met Asp Leu Glu Leu Val Leu Met Cys Lys
                85                  90                  95
Asp Lys Pro Thr Glu Thr Leu Leu Asn Thr Val Lys Asp Asn Leu Pro
            100                 105                 110
Ile Gln Ile Gln Lys Leu Thr Glu Glu Lys Tyr Gln Val Glu Gln Cys
        115                 120                 125
Val Asn Glu Ala Ser Ile Ile Ile Arg Asn Thr Lys Glu Pro Thr Leu
    130                 135                 140
Thr Leu Lys Val Ile Leu Thr Ser Pro Leu Ile Arg Asp Glu Leu Glu
145                 150                 155                 160
Lys Lys Asp Gly Glu Asn Val Ser Met Lys Asp Pro Pro Asp Leu Leu
                165                 170                 175
Asp Arg Gln Lys Cys Leu Asn Ala Leu Ala Ser Leu Arg His Ala Lys
            180                 185                 190
Trp Phe Gln Ala Arg Ala Asn Gly Leu Lys Ser Cys Val Ile Val Leu
        195                 200                 205
Arg Ile Leu Arg Asp Leu Cys Asn Arg Val Pro Thr Trp Ala Pro Leu
    210                 215                 220
Lys Gly Trp Pro Leu Glu Leu Ile Cys Glu Lys Ser Ile Gly Thr Cys
225                 230                 235                 240
Asn Arg Pro Leu Gly Ala Gly Glu Ala Leu Arg Arg Val Met Glu Cys
                245                 250                 255
Leu Ala Ser Gly Ile Leu Leu Pro Gly Gly Pro Gly Leu His Asp Pro
            260                 265                 270
Cys Glu Arg Asp Pro Thr Asp Ala Leu Ser Tyr Met Thr Ile Gln Gln
        275                 280                 285
Lys Glu Asp Ile Thr His Ser Ala Gln His Ala Leu Arg Leu Ser Ala
    290                 295                 300
Phe Gly Gln Ile Tyr Lys Val Leu Glu Met Asp Pro Leu Pro Ser Ser
305                 310                 315                 320
Lys Pro Phe Gln Lys Tyr Ser Trp Ser Val Thr Asp Lys Glu Gly Ala
                325                 330                 335
Gly Ser Ser Ala Leu Lys Arg Pro Phe Glu Asp Gly Leu Gly Asp Asp
            340                 345                 350
Lys Asp Pro Asn Lys Lys Met Lys Arg Asn Leu Arg Lys Ile Leu Asp
        355                 360                 365
Ser Lys Ala Ile Asp Leu Met Asn Ala Leu Met Arg Leu Asn Gln Ile
    370                 375                 380
Arg Pro Gly Leu Gln Tyr Lys Leu Leu Ser Gln Ser Gly Pro Val His
385                 390                 395                 400
Ala Pro Val Phe Thr Met Ser Val Asp Val Asp Gly Thr Thr Tyr Glu
                405                 410                 415

Ala Ser Gly Pro Ser Lys Lys Thr Ala Lys Leu His Val Ala Val Lys
            420                 425                 430
Val Leu Gln Ala Met Gly Tyr Pro Thr Gly Phe Asp Ala Asp Ile Glu
        435                 440                 445
Cys Met Ser Ser Asp Glu Lys Ser Asp Asn Glu Ser Lys Asn Glu Thr
    450                 455                 460
Val Ser Ser Asn Ser Ser Asn Asn Thr Gly Asn Ser Thr Thr Glu Thr
465                 470                 475                 480
Ser Ser Thr Leu Glu Val Arg Thr Gln Gly Pro Ile Leu Thr Ala Ser
                485                 490                 495
Gly Lys Asn Pro Val Met Glu Leu Asn Glu Lys Arg Arg Gly Leu Lys
            500                 505                 510
Tyr Glu Leu Ile Ser Glu Thr Gly Gly Ser His Asp Lys Arg Phe Val
        515                 520                 525
Met Glu Val Glu Val Asp Gly Gln Lys Phe Arg Gly Ala Gly Pro Asn
    530                 535                 540
Lys Lys Val Ala Lys Ala Ser Ala Ala Leu Ala Ala Leu Glu Lys Leu
545                 550                 555                 560
Phe Ser Gly Pro Asn Ala Ala Asn Asn Lys Lys Lys Lys Ile Ile Pro
                565                 570                 575
Gln Ala Lys Gly Val Val Asn Thr Ala Val Ser Ala Ala Val Gln Ala
            580                 585                 590
Val Arg Gly Arg Gly Arg Gly Thr Leu Thr Arg Gly Ala Phe Val Gly
        595                 600                 605
Ala Thr Ala Ala Pro Gly Tyr Ile Ala Pro Gly Tyr Gly Thr Pro Tyr
    610                 615                 620
Gly Tyr Ser Thr Ala Ala Pro Ala Tyr Gly Leu Pro Lys Arg Met Val
625                 630                 635                 640
Leu Leu Pro Val Met Lys Phe Pro Thr Tyr Pro Val Pro His Tyr Ser
                645                 650                 655
Phe Phe

<210> 6
<211> 405
<212> PRT
<213> Homo sapiens
<400> 6
Met Glu Cys Leu Ala Ser Gly Ile Leu Leu Pro Gly Gly Pro Gly Leu
1               5                   10                  15
His Asp Pro Cys Glu Arg Asp Pro Thr Asp Ala Leu Ser Tyr Met Thr
            20                  25                  30
Ile Gln Gln Lys Glu Asp Ile Thr His Ser Ala Gln His Ala Leu Arg
        35                  40                  45
Leu Ser Ala Phe Gly Gln Ile Tyr Lys Val Leu Glu Met Asp Pro Leu
    50                  55                  60

Pro Ser Ser Lys Pro Phe Gln Lys Tyr Ser Trp Ser Val Thr Asp Lys
65                  70                  75                  80
Glu Gly Ala Gly Ser Ser Ala Leu Lys Arg Pro Phe Glu Asp Gly Leu
                85                  90                  95
Gly Asp Asp Lys Asp Pro Asn Lys Lys Met Lys Arg Asn Leu Arg Lys
            100                 105                 110
Ile Leu Asp Ser Lys Ala Ile Asp Leu Met Asn Ala Leu Met Arg Leu
        115                 120                 125
Asn Gln Ile Arg Pro Gly Leu Gln Tyr Lys Leu Leu Ser Gln Ser Gly
    130                 135                 140
Pro Val His Ala Pro Val Phe Thr Met Ser Val Asp Val Asp Gly Thr
145                 150                 155                 160
Thr Tyr Glu Ala Ser Gly Pro Ser Lys Lys Thr Ala Lys Leu His Val
                165                 170                 175
Ala Val Lys Val Leu Gln Ala Met Gly Tyr Pro Thr Gly Phe Asp Ala
            180                 185                 190
Asp Ile Glu Cys Met Ser Ser Asp Glu Lys Ser Asp Asn Glu Ser Lys
        195                 200                 205
Asn Glu Thr Val Ser Ser Asn Ser Ser Asn Asn Thr Gly Asn Ser Thr
    210                 215                 220
Thr Glu Thr Ser Ser Thr Leu Glu Val Arg Thr Gln Gly Pro Ile Leu
225                 230                 235                 240
Thr Ala Ser Gly Lys Asn Pro Val Met Glu Leu Asn Glu Lys Arg Arg
                245                 250                 255
Gly Leu Lys Tyr Glu Leu Ile Ser Glu Thr Gly Gly Ser His Asp Lys
            260                 265                 270
Arg Phe Val Met Glu Val Glu Val Asp Gly Gln Lys Phe Arg Gly Ala
        275                 280                 285
Gly Pro Asn Lys Lys Val Ala Lys Ala Ser Ala Ala Leu Ala Ala Leu
    290                 295                 300
Glu Lys Leu Phe Ser Gly Pro Asn Ala Ala Asn Asn Lys Lys Lys Lys
305                 310                 315                 320
Ile Ile Pro Gln Ala Lys Gly Val Val Asn Thr Ala Val Ser Ala Ala
                325                 330                 335
Val Gln Ala Val Arg Gly Arg Gly Arg Gly Thr Leu Thr Arg Gly Ala
            340                 345                 350
Phe Val Gly Ala Thr Ala Ala Pro Gly Tyr Ile Ala Pro Gly Tyr Gly
        355                 360                 365
Thr Pro Tyr Gly Tyr Ser Thr Ala Ala Pro Ala Tyr Gly Leu Pro Lys
    370                 375                 380
Arg Met Val Leu Leu Pro Val Met Lys Phe Pro Thr Tyr Pro Val Pro
385                 390                 395                 400
His Tyr Ser Phe Phe
                405

<210> 7

<211> 702
<212> PRT
<213> Homo sapiens
<400> 7

<210> 8
<211> 764
<212> PRT
<213> Homo sapiens
<400> 8
Met Arg Pro Met Arg Ile Phe Val Asn Asp Asp Arg His Val Met Ala
1               5                   10                  15
Lys His Ser Ser Val Tyr Pro Thr Gln Glu Glu Leu Glu Ala Val Gln
            20                  25                  30
Asn Met Val Ser His Thr Glu Arg Ala Leu Lys Ala Val Ser Asp Trp
        35                  40                  45
Ile Asp Glu Gln Glu Lys Gly Ser Ser Glu Gln Ala Glu Ser Asp Asn
    50                  55                  60
Met Asp Val Pro Pro Glu Asp Asp Ser Lys Glu Gly Ala Gly Glu Gln
65                  70                  75                  80
Lys Thr Glu His Met Thr Arg Thr Leu Arg Gly Val Met Arg Val Gly
                85                  90                  95
Leu Val Ala Lys Gly Leu Leu Leu Lys Gly Asp Leu Asp Leu Glu Leu
            100                 105                 110
Val Leu Leu Cys Lys Glu Lys Pro Thr Thr Ala Leu Leu Asp Lys Val
        115                 120                 125
Ala Asp Asn Leu Ala Ile Gln Leu Ala Ala Val Thr Glu Asp Lys Tyr
    130                 135                 140
Glu Ile Leu Gln Ser Val Asp Asp Ala Ala Ile Val Ile Lys Asn Thr
145                 150                 155                 160
Lys Glu Pro Pro Leu Ser Leu Thr Ile His Leu Thr Ser Pro Val Val
                165                 170                 175
Arg Glu Glu Met Glu Lys Val Leu Ala Gly Glu Thr Leu Ser Val Asn
            180                 185                 190
Asp Pro Pro Asp Val Leu Asp Arg Gln Lys Cys Leu Ala Ala Leu Ala
        195                 200                 205
Ser Leu Arg His Ala Lys Trp Phe Gln Ala Arg Ala Asn Gly Leu Lys
    210                 215                 220
Ser Cys Val Ile Val Ile Arg Val Leu Arg Asp Leu Cys Thr Arg Val
225                 230                 235                 240
Pro Thr Trp Gly Pro Leu Arg Gly Trp Pro Leu Glu Leu Leu Cys Glu
                245                 250                 255
Lys Ser Ile Gly Thr Ala Asn Arg Pro Met Gly Ala Gly Glu Ala Leu
            260                 265                 270
Arg Arg Val Leu Glu Cys Leu Ala Ser Gly Ile Val Met Pro Asp Gly
        275                 280                 285
Ser Gly Ile Tyr Asp Pro Cys Glu Lys Glu Ala Thr Asp Ala Ile Gly
    290                 295                 300
His Leu Asp Arg Gln Gln Arg Glu Asp Ile Thr Gln Ser Ala Gln His
305                 310                 315                 320

Ala Leu Arg Leu Ala Ala Phe Gly Gln Leu His Lys Val Leu Gly Met
                325                 330                 335
Asp Pro Leu Pro Ser Lys Met Pro Lys Lys Pro Lys Asn Glu Asn Pro
            340                 345                 350
Val Asp Tyr Thr Val Gln Ile Pro Pro Ser Thr Thr Tyr Ala Ile Thr
        355                 360                 365
Pro Met Lys Arg Pro Met Glu Glu Asp Gly Glu Glu Lys Ser Pro Ser
    370                 375                 380
Lys Lys Lys Lys Lys Ile Gln Lys Lys Glu Glu Lys Ala Glu Pro Pro
385                 390                 395                 400
Gln Ala Met Asn Ala Leu Met Arg Leu Asn Gln Leu Lys Pro Gly Leu
                405                 410                 415
Gln Tyr Lys Leu Val Ser Gln Thr Gly Pro Val His Ala Pro Ile Phe
            420                 425                 430
Thr Met Ser Val Glu Val Asp Gly Asn Ser Phe Glu Ala Ser Gly Pro
        435                 440                 445
Ser Lys Lys Thr Ala Lys Leu His Val Ala Val Lys Val Leu Gln Asp
    450                 455                 460
Met Gly Leu Pro Thr Gly Ala Glu Gly Arg Asp Ser Ser Lys Gly Glu
465                 470                 475                 480
Asp Ser Ala Glu Glu Thr Glu Ala Lys Pro Ala Val Val Ala Pro Ala
                485                 490                 495
Pro Val Val Glu Ala Val Ser Thr Pro Ser Ala Ala Phe Pro Ser Asp
            500                 505                 510
Ala Thr Ala Glu Gln Gly Pro Ile Leu Thr Lys His Gly Lys Asn Pro
        515                 520                 525
Val Met Glu Leu Asn Glu Lys Arg Arg Gly Leu Lys Tyr Glu Leu Ile
    530                 535                 540
Ser Glu Thr Gly Gly Ser His Asp Lys Arg Phe Val Met Glu Val Glu
545                 550                 555                 560
Val Asp Gly Gln Lys Phe Gln Gly Ala Gly Ser Asn Lys Lys Val Ala
                565                 570                 575
Lys Ala Tyr Ala Ala Leu Ala Ala Leu Glu Lys Leu Phe Pro Asp Thr
            580                 585                 590
Pro Leu Ala Leu Asp Ala Asn Lys Lys Lys Arg Ala Pro Val Pro Val
        595                 600                 605
Arg Gly Gly Pro Lys Phe Ala Ala Lys Pro His Asn Pro Gly Phe Gly
    610                 615                 620
Met Gly Gly Pro Met His Asn Glu Val Pro Pro Pro Pro Asn Leu Arg
625                 630                 635                 640
Gly Arg Gly Arg Gly Gly Ser Ile Arg Gly Arg Gly Arg Gly Arg Gly
                645                 650                 655
Phe Gly Gly Ala Asn His Gly Gly Tyr Met Asn Ala Gly Ala Gly Tyr
            660                 665                 670
Gly Ser Tyr Gly Tyr Gly Gly Asn Ser Ala Thr Ala Gly Tyr Ser Gln
        675                 680                 685

Phe Ser Arg Pro Pro Pro Pro Ser Arg Pro Arg Cys Cys Val Val Arg
    690                 695                 700
Cys Ser Gly Ser Pro Cys Gly Pro Ser Cys Asp Pro Tyr Leu Ala Val
705                 710                 715                 720
Phe Gly Thr Pro Cys Leu Gln Trp Phe Val Ser Cys His Tyr Asn Phe
                725                 730                 735
Val Trp Val Glu Phe Leu Ser Phe Cys Ser Ser Val Ser Leu Cys Leu
            740                 745                 750
Phe Thr Leu Arg Val Ser Gly Asn Ser Val Cys Leu
        755                 760

<210> 9
<211> 702
<212> PRT
<213> Homo sapiens
<400> 9
Met Arg Pro Met Arg Ile Phe Val Asn Asp Asp Arg His Val Met Ala
  1               5                  10                  15
Lys His Ser Ser Val Tyr Pro Thr Gln Glu Glu Leu Glu Ala Val Gln
             20                  25                  30
Asn Met Val Ser His Thr Glu Arg Ala Leu Lys Ala Val Ser Asp Trp
         35                  40                  45
Ile Asp Glu Gln Glu Lys Gly Ser Ser Glu Gln Ala Glu Ser Asp Asn
     50                  55                  60
Met Asp Val Pro Pro Glu Asp Asp Ser Lys Glu Gly Ala Gly Glu Gln
 65                  70                  75                  80
Lys Thr Glu His Met Thr Arg Thr Leu Arg Gly Val Met Arg Val Gly
                 85                  90                  95
Leu Val Ala Lys Gly Leu Leu Leu Lys Gly Asp Leu Asp Leu Glu Leu
            100                 105                 110
Val Leu Leu Cys Lys Glu Lys Pro Thr Thr Ala Leu Leu Asp Lys Val
        115                 120                 125
Ala Asp Asn Leu Ala Ile Gln Leu Ala Ala Val Thr Glu Asp Lys Tyr
    130                 135                 140
Glu Ile Leu Gln Ser Val Asp Asp Ala Ala Ile Val Ile Lys Asn Thr
145                 150                 155                 160
Lys Glu Pro Pro Leu Ser Leu Thr Ile His Leu Thr Ser Pro Val Val
                165                 170                 175
Arg Glu Glu Met Glu Lys Val Leu Ala Gly Glu Thr Leu Ser Val Asn
            180                 185                 190
Asp Pro Pro Asp Val Leu Asp Arg Gln Lys Cys Leu Ala Ala Leu Ala
        195                 200                 205
Ser Leu Arg His Ala Lys Trp Phe Gln Ala Arg Ala Asn Gly Leu Lys
    210                 215                 220
Ser Cys Val Ile Val Ile Arg Val Leu Arg Asp Leu Cys Thr Arg Val
225                 230                 235                 240

Pro Thr Trp Gly Pro Leu Arg Gly Trp Pro Leu Glu Leu Leu Cys Glu
                245                 250                 255
Lys Ser Ile Gly Thr Ala Asn Arg Pro Met Gly Ala Gly Glu Ala Leu
            260                 265                 270
Arg Arg Val Leu Glu Cys Leu Ala Ser Gly Ile Val Met Pro Asp Gly
        275                 280                 285
Ser Gly Ile Tyr Asp Pro Cys Glu Lys Glu Ala Thr Asp Ala Ile Gly
    290                 295                 300
His Leu Asp Arg Gln Gln Arg Glu Asp Ile Thr Gln Ser Ala Gln His
305                 310                 315                 320
Ala Leu Arg Leu Ala Ala Phe Gly Gln Leu His Lys Val Leu Gly Met
                325                 330                 335
Asp Pro Leu Pro Ser Lys Met Pro Lys Lys Pro Lys Asn Glu Asn Pro
            340                 345                 350
Val Asp Tyr Thr Val Gln Ile Pro Pro Ser Thr Thr Tyr Ala Ile Thr
        355                 360                 365
Pro Met Lys Arg Pro Met Glu Glu Asp Gly Glu Glu Lys Ser Pro Ser
    370                 375                 380
Lys Lys Lys Lys Lys Ile Gln Lys Lys Glu Glu Lys Ala Glu Pro Pro
385                 390                 395                 400
Gln Ala Met Asn Ala Leu Met Arg Leu Asn Gln Leu Lys Pro Gly Leu
                405                 410                 415
Gln Tyr Lys Leu Val Ser Gln Thr Gly Pro Val His Ala Pro Ile Phe
            420                 425                 430
Thr Met Ser Val Glu Val Asp Gly Asn Ser Phe Glu Ala Ser Gly Pro
        435                 440                 445
Ser Lys Lys Thr Ala Lys Leu His Val Ala Val Lys Val Leu Gln Asp
    450                 455                 460
Met Gly Leu Pro Thr Gly Ala Glu Gly Arg Asp Ser Ser Lys Gly Glu
465                 470                 475                 480
Asp Ser Ala Glu Glu Thr Glu Ala Lys Pro Ala Val Val Ala Pro Ala
                485                 490                 495
Pro Val Val Glu Ala Val Ser Thr Pro Ser Ala Ala Phe Pro Ser Asp
            500                 505                 510
Ala Thr Ala Glu Gln Gly Pro Ile Leu Thr Lys His Gly Lys Asn Pro
        515                 520                 525
Val Met Glu Leu Asn Glu Lys Arg Arg Gly Leu Lys Tyr Glu Leu Ile
    530                 535                 540
Ser Glu Thr Gly Gly Ser His Asp Lys Arg Phe Val Met Glu Val Glu
545                 550                 555                 560
Val Asp Gly Gln Lys Phe Gln Gly Ala Gly Ser Asn Lys Lys Val Ala
                565                 570                 575
Lys Ala Tyr Ala Ala Leu Ala Ala Leu Glu Lys Leu Phe Pro Asp Thr
            580                 585                 590
Pro Leu Ala Leu Asp Ala Asn Lys Lys Lys Arg Ala Pro Val Pro Val
        595                 600                 605

Arg Gly Gly Pro Lys Phe Ala Ala Lys Pro His Asn Pro Gly Phe Gly
    610                 615                 620
Met Gly Gly Pro Met His Asn Glu Val Pro Pro Pro Pro Asn Leu Arg
625                 630                 635                 640
Gly Arg Gly Arg Gly Gly Ser Ile Arg Gly Arg Gly Arg Gly Arg Gly
                645                 650                 655
Phe Gly Gly Ala Asn His Gly Gly Tyr Met Asn Ala Gly Ala Gly Tyr
            660                 665                 670
Gly Ser Tyr Gly Tyr Gly Gly Asn Ser Ala Thr Ala Gly Tyr Ser Asp
        675                 680                 685
Phe Phe Thr Asp Cys Tyr Gly Tyr His Asp Phe Gly Ser Ser
    690                 695                 700

<210> 10
<211> 690
<212> PRT
<213> Homo sapiens
<400> 10
Met Arg Pro Met Arg Ile Phe Val Asn Asp Asp Arg His Val Met Ala
1               5                   10                  15
Lys His Ser Ser Val Tyr Pro Thr Gln Glu Glu Leu Glu Ala Val Gln
            20                  25                  30
Asn Met Val Ser His Thr Glu Arg Ala Leu Lys Ala Val Ser Asp Trp
        35                  40                  45
Ile Asp Glu Gln Glu Lys Gly Ser Ser Glu Gln Ala Glu Ser Asp Asn
    50                  55                  60
Met Asp Val Pro Pro Glu Asp Asp Ser Lys Glu Gly Ala Gly Glu Gln
65                  70                  75                  80
Lys Thr Glu His Met Thr Arg Thr Leu Arg Gly Val Met Arg Val Gly
                85                  90                  95
Leu Val Ala Lys Gly Leu Leu Leu Lys Gly Asp Leu Asp Leu Glu Leu
            100                 105                 110
Val Leu Leu Cys Lys Glu Lys Pro Thr Thr Ala Leu Leu Asp Lys Val
        115                 120                 125
Ala Asp Asn Leu Ala Ile Gln Leu Ala Ala Val Thr Glu Asp Lys Tyr
    130                 135                 140
Glu Ile Leu Gln Ser Val Asp Asp Ala Ala Ile Val Ile Lys Asn Thr
145                 150                 155                 160
Lys Glu Pro Pro Leu Ser Leu Thr Ile His Leu Thr Ser Pro Val Val
                165                 170                 175
Arg Glu Glu Met Glu Lys Val Leu Ala Gly Glu Thr Leu Ser Val Asn
            180                 185                 190
Asp Pro Pro Asp Val Leu Asp Arg Gln Lys Cys Leu Ala Ala Leu Ala
        195                 200                 205
Ser Leu Arg His Ala Lys Trp Phe Gln Ala Arg Ala Asn Gly Leu Lys
    210                 215                 220

Ser Cys Val Ile Val Ile Arg Val Leu Arg Asp Leu Cys Thr Arg Val
225                 230                 235                 240
Pro Thr Trp Gly Pro Leu Arg Gly Trp Pro Leu Glu Leu Leu Cys Glu
                245                 250                 255
Lys Ser Ile Gly Thr Ala Asn Arg Pro Met Gly Ala Gly Glu Ala Leu
            260                 265                 270
Arg Arg Val Leu Glu Cys Leu Ala Ser Gly Ile Val Met Pro Asp Gly
        275                 280                 285
Ser Gly Ile Tyr Asp Pro Cys Glu Lys Glu Ala Thr Asp Ala Ile Gly
    290                 295                 300
His Leu Asp Arg Gln Gln Arg Glu Asp Ile Thr Gln Ser Ala Gln His
305                 310                 315                 320
Ala Leu Arg Leu Ala Ala Phe Gly Gln Leu His Lys Val Leu Gly Met
                325                 330                 335
Asp Pro Leu Pro Ser Lys Met Pro Lys Lys Pro Lys Asn Glu Asn Pro
            340                 345                 350
Val Asp Tyr Thr Val Gln Ile Pro Pro Ser Thr Thr Tyr Ala Ile Thr
        355                 360                 365
Pro Met Lys Arg Pro Met Glu Glu Asp Gly Glu Glu Lys Ser Pro Ser
    370                 375                 380
Lys Lys Lys Lys Lys Ile Gln Lys Lys Glu Glu Lys Ala Glu Pro Pro
385                 390                 395                 400
Gln Ala Met Asn Ala Leu Met Arg Leu Asn Gln Leu Lys Pro Gly Leu
                405                 410                 415
Gln Tyr Lys Leu Val Ser Gln Thr Gly Pro Val His Ala Pro Ile Phe
            420                 425                 430
Thr Met Ser Val Glu Val Asp Gly Asn Ser Phe Glu Ala Ser Gly Pro
        435                 440                 445
Ser Lys Lys Thr Ala Lys Leu His Val Ala Val Lys Val Leu Gln Asp
    450                 455                 460
Met Gly Leu Pro Thr Gly Ala Glu Gly Arg Asp Ser Ser Lys Gly Glu
465                 470                 475                 480
Asp Ser Ala Glu Glu Thr Glu Ala Lys Pro Ala Val Val Ala Pro Ala
                485                 490                 495
Pro Val Val Glu Ala Val Ser Thr Pro Ser Ala Ala Phe Pro Ser Asp
            500                 505                 510
Ala Thr Ala Glu Gln Gly Pro Ile Leu Thr Lys His Gly Lys Asn Pro
        515                 520                 525
Val Met Glu Leu Asn Glu Lys Arg Arg Gly Leu Lys Tyr Glu Leu Ile
    530                 535                 540
Ser Glu Thr Gly Gly Ser His Asp Lys Arg Phe Val Met Glu Val Glu
545                 550                 555                 560
Val Asp Gly Gln Lys Phe Gln Gly Ala Gly Ser Asn Lys Lys Val Ala
                565                 570                 575
Lys Ala Tyr Ala Ala Leu Ala Ala Leu Glu Lys Leu Phe Pro Asp Thr
            580                 585                 590

Pro Leu Ala Leu Asp Ala Asn Lys Lys Lys Arg Ala Pro Val Pro Val
        595                 600                 605
Arg Gly Gly Pro Lys Phe Ala Ala Lys Pro His Asn Pro Gly Phe Gly
    610                 615                 620
Met Gly Gly Pro Met His Asn Glu Val Pro Pro Pro Pro Asn Leu Arg
625                 630                 635                 640
Gly Arg Gly Arg Gly Gly Ser Ile Arg Gly Arg Gly Arg Gly Arg Gly
                645                 650                 655
Phe Gly Gly Ala Asn His Gly Gly Tyr Met Asn Ala Gly Ala Gly Tyr
            660                 665                 670
Gly Ser Tyr Gly Tyr Gly Gly Asn Ser Ala Thr Ala Gly Tyr Thr Gly
        675                 680                 685
Phe Val
    690

<210> 11
<211> 894
<212> PRT
<213> Homo sapiens
<400> 11

<210> 12
<211> 564
<212> PRT
<213> Homo sapiens
<400> 12

Gln Leu Ala Ala Val Thr Glu Asp Lys Tyr Glu Ile Leu Gln Ser Val
1               5                   10                  15
Asp Asp Ala Ala Ile Val Ile Lys Asn Thr Lys Glu Pro Pro Leu Ser
            20                  25                  30
Leu Thr Ile His Leu Thr Ser Pro Val Val Arg Glu Glu Met Glu Lys
        35                  40                  45
Val Leu Ala Gly Glu Thr Leu Ser Val Asn Asp Pro Pro Asp Val Leu
    50                  55                  60
Asp Arg Gln Lys Cys Leu Ala Ala Leu Ala Ser Leu Arg His Ala Lys
65                  70                  75                  80
Trp Phe Gln Ala Arg Ala Asn Gly Leu Lys Ser Cys Val Ile Val Ile
                85                  90                  95
Arg Val Leu Arg Asp Leu Cys Thr Arg Val Pro Thr Trp Gly Pro Leu
            100                 105                 110
Arg Gly Trp Pro Leu Glu Leu Leu Cys Glu Lys Ser Ile Gly Thr Ala
        115                 120                 125
Asn Arg Pro Met Gly Ala Gly Glu Ala Leu Arg Arg Val Leu Glu Cys
    130                 135                 140
Leu Ala Ser Gly Ile Val Met Pro Asp Gly Ser Gly Ile Tyr Asp Pro
145                 150                 155                 160
Cys Glu Lys Glu Ala Thr Asp Ala Ile Gly His Leu Asp Arg Gln Gln
                165                 170                 175
Arg Glu Asp Ile Thr Gln Ser Ala Gln His Ala Leu Arg Leu Ala Ala
            180                 185                 190
Phe Gly Gln Leu His Lys Val Leu Gly Met Asp Pro Leu Pro Ser Lys
        195                 200                 205
Met Pro Lys Lys Pro Lys Asn Glu Asn Pro Val Asp Tyr Thr Val Gln
    210                 215                 220
Ile Pro Pro Ser Thr Thr Tyr Ala Ile Thr Pro Met Lys Arg Pro Met
225                 230                 235                 240
Glu Glu Asp Gly Glu Glu Lys Ser Pro Ser Lys Lys Lys Lys Lys Ile
                245                 250                 255
Gln Lys Lys Glu Glu Lys Ala Glu Pro Pro Gln Ala Met Asn Ala Leu
            260                 265                 270
Met Arg Leu Asn Gln Leu Lys Pro Gly Leu Gln Tyr Lys Leu Val Ser
        275                 280                 285
Gln Thr Gly Pro Val His Ala Pro Ile Phe Thr Met Ser Val Glu Val
    290                 295                 300
Asp Gly Asn Ser Phe Glu Ala Ser Gly Pro Ser Lys Lys Thr Ala Lys
305                 310                 315                 320
Leu His Val Ala Val Lys Val Leu Gln Asp Met Gly Leu Pro Thr Gly
                325                 330                 335
Ala Glu Gly Arg Asp Ser Ser Lys Gly Glu Asp Ser Ala Glu Glu Thr
            340                 345                 350
Glu Ala Lys Pro Ala Val Val Ala Pro Ala Pro Val Val Glu Ala Val
        355                 360                 365

Ser Thr Pro Ser Ala Ala Phe Pro Ser Asp Ala Thr Ala Glu Asn Val
    370                 375                 380
Lys Gln Gln Gly Pro Ile Leu Thr Lys His Gly Lys Asn Pro Val Met
385                 390                 395                 400
Glu Leu Asn Glu Lys Arg Arg Gly Leu Lys Tyr Glu Leu Ile Ser Glu
                405                 410                 415
Thr Gly Gly Ser His Asp Lys Arg Phe Val Met Glu Val Glu Val Asp
            420                 425                 430
Gly Gln Lys Phe Gln Gly Ala Gly Ser Asn Lys Lys Val Ala Lys Ala
        435                 440                 445
Tyr Ala Ala Leu Ala Ala Leu Glu Lys Leu Phe Pro Asp Thr Pro Leu
    450                 455                 460
Ala Leu Asp Ala Asn Lys Lys Lys Arg Ala Pro Val Pro Val Arg Gly
465                 470                 475                 480
Gly Pro Lys Phe Ala Ala Lys Pro His Asn Pro Gly Phe Gly Met Gly
                485                 490                 495
Gly Pro Met His Asn Glu Val Pro Pro Pro Pro Asn Leu Arg Gly Arg
            500                 505                 510
Gly Arg Gly Gly Ser Ile Arg Gly Arg Gly Arg Gly Arg Gly Phe Gly
        515                 520                 525
Gly Ala Asn His Gly Gly Tyr Met Asn Ala Gly Ala Gly Tyr Gly Ser
    530                 535                 540
Tyr Gly Tyr Gly Gly Asn Ser Ala Thr Ala Gly Tyr Ser Lys Cys Ala
545                 550                 555                 560
Phe Leu Ser Val

<210> 13
<211> 671
<212> PRT
<213> Homo sapiens
<400> 13
Met Arg Pro Met Arg Ile Phe Val Asn Asp Asp Arg His Val Met Ala
1               5                   10                  15
Lys His Ser Ser Val Tyr Pro Thr Gln Glu Glu Leu Glu Ala Val Gln
            20                  25                  30
Asn Met Val Ser His Thr Glu Arg Ala Leu Lys Ala Val Ser Asp Trp
        35                  40                  45
Ile His Glu Gln Glu Lys Gly Ser Ser Glu Gln Ala Glu Ser Asp Asn
    50                  55                  60
Met Asp Val Pro Pro Glu Asp Asp Ser Lys Glu Gly Ala Gly Glu Gln
65                  70                  75                  80
Lys Thr Glu His Met Thr Arg Thr Cys Arg Gly Val Met Arg Ala Gly
                85                  90                  95
Pro Gly Gly Gln Ser Ala Ser Tyr Ser Arg Gly Thr Trp Ile Trp Ser
            100                 105                 110

Trp Cys Cys Cys Val Arg Arg Ser Pro Gln Pro Ala Leu Leu Asp Lys
        115                 120                 125
Val Ala Asp Asn Leu Ala Ile Gln Leu Ala Ala Val Thr Glu Asp Lys
    130                 135                 140
Tyr Glu Ile Leu Gln Ser Val Asp Asp Ala Ala Ile Val Ile Lys Asn
145                 150                 155                 160
Thr Lys Glu Pro Pro Leu Ser Leu Thr Ile His Leu Thr Ser Pro Val
                165                 170                 175
Val Arg Glu Glu Met Glu Lys Val Leu Ala Gly Glu Thr Leu Ser Val
            180                 185                 190
Asn Asp Pro Pro Asp Val Leu Asp Arg Gln Lys Cys Phe Ala Ala Leu
        195                 200                 205
Ala Ser Leu Arg His Ala Lys Trp Phe Gln Ala Arg Ala Asn Gly Leu
    210                 215                 220
Lys Ser Cys Val Ile Val Ile Arg Val Leu Arg Asp Leu Cys Thr Arg
225                 230                 235                 240
Val Pro Thr Trp Gly Pro Leu Arg Gly Trp Pro Leu Glu Leu Leu Cys
                245                 250                 255
Glu Lys Ser Ile Gly Thr Ala Asn Arg Pro Met Gly Ala Gly Glu Ala
            260                 265                 270
Leu Arg Arg Val Leu Glu Cys Leu Ala Ser Gly Ile Val Met Pro Asp
        275                 280                 285
Gly Ser Gly Ile Tyr Asp Pro Cys Glu Lys Glu Ala Thr Asp Ala Ile
    290                 295                 300
Gly His Leu Asp Arg Gln Gln Arg Glu Asp Ile Thr Gln Ser Ala Gln
305                 310                 315                 320
His Ala Leu Arg Leu Ala Ala Phe Gly Gln Leu His Lys Val Leu Gly
                325                 330                 335
Met Asp Pro Leu Pro Ser Lys Met Pro Lys Lys Pro Lys Asn Glu Asn
            340                 345                 350
Pro Val Asp Tyr Thr Val Gln Ile Pro Pro Ser Thr Thr Tyr Ala Ile
        355                 360                 365
Thr Pro Met Lys Arg Pro Met Glu Glu Asp Gly Glu Glu Lys Ser Pro
    370                 375                 380
Ser Lys Lys Lys Lys Lys Ile Gln Lys Lys Glu Glu Lys Ala Glu Pro
385                 390                 395                 400
Pro Gln Ala Met Asn Ala Leu Met Arg Leu Asn Gln Leu Lys Pro Gly
                405                 410                 415
Leu Gln Tyr Lys Leu Val Ser Gln Thr Gly Pro Val His Ala Pro Ile
            420                 425                 430
Phe Thr Met Ser Val Glu Val Asp Gly Asn Ser Phe Glu Ala Ser Gly
        435                 440                 445
Pro Ser Lys Lys Thr Ala Lys Leu His Val Ala Val Lys Val Leu Gln
    450                 455                 460
Asp Met Gly Leu Pro Thr Gly Ala Glu Gly Arg Asp Ser Ser Lys Gly
465                 470                 475                 480

Glu Asp Ser Ala Glu Glu Thr Glu Ala Lys Pro Ala Val Val Ala Pro
                485                 490                 495
Ala Pro Val Val Glu Ala Val Ser Thr Pro Ser Ala Ala Phe Pro Ser
            500                 505                 510
Asp Ala Thr Ala Glu Asn Val Lys Gln Gln Gly Pro Ile Leu Thr Lys
        515                 520                 525
His Gly Lys Asn Pro Val Met Glu Leu Asn Glu Lys Arg Arg Gly Leu
    530                 535                 540
Lys Tyr Glu Leu Ile Ser Glu Thr Gly Gly Ser His Asp Lys Arg Phe
545                 550                 555                 560
Val Met Glu Val Glu Val Asp Gly Gln Lys Phe Gln Gly Ala Gly Ser
                565                 570                 575
Asn Lys Lys Val Ala Lys Ala Tyr Ala Ala Leu Ala Ala Leu Glu Lys
            580                 585                 590
Leu Phe Pro Asp Thr Pro Leu Ser Pro Leu Met Pro Thr Lys Arg Arg
        595                 600                 605
Glu Pro Gln Tyr Pro Ser Glu Gly Asp Arg Asn Leu Leu Leu Ser His
    610                 615                 620
Ile Thr Leu Ala Ser Ala Trp Glu Ala Pro Cys Thr Thr Lys Cys Pro
625                 630                 635                 640
His Pro Pro Thr Phe Glu Gly Gly Glu Glu Ala Gly Arg Ser Gly Asp
                645                 650                 655
Glu Gly Ala Gly Glu Asp Leu Val Ala Pro Thr Met Glu Ala Thr
            660                 665                 670

<210> 14
<211> 611
<212> PRT
<213> Homo sapiens
<400> 14
Met Arg Pro Met Arg Ile Phe Val Asn Asp Asp Arg His Val Met Ala
1               5                   10                  15
Lys His Ser Ser Val Tyr Pro Thr Gln Glu Glu Leu Glu Ala Val Gln
            20                  25                  30
Asn Met Val Ser His Thr Glu Arg Ala Leu Lys Ala Val Ser Asp Trp
        35                  40                  45
Ile Asp Glu Gln Glu Lys Gly Ser Ser Glu Gln Ala Glu Ser Asp Asn
    50                  55                  60
Met Asp Val Pro Pro Glu Asp Asp Ser Lys Glu Gly Ala Gly Glu Gln
65                  70                  75                  80
Lys Thr Glu His Met Thr Arg Thr Leu Arg Gly Val Met Arg Val Gly
                85                  90                  95
Leu Val Ala Lys Gly Leu Leu Leu Lys Gly Asp Leu Asp Leu Glu Leu
            100                 105                 110
Val Leu Leu Cys Lys Glu Lys Pro Thr Thr Ala Leu Leu Asp Lys Val
        115                 120                 125

Ala Asp Asn Leu Ala Ile Gln Leu Ala Ala Val Thr Glu Asp Lys Tyr
    130                 135                 140
Glu Ile Leu Gln Ser Val Asp Asp Ala Ala Ile Val Ile Lys Asn Thr
145                 150                 155                 160
Lys Glu Pro Pro Leu Ser Leu Thr Ile His Leu Thr Ser Pro Val Val
                165                 170                 175
Arg Glu Glu Met Glu Lys Val Leu Ala Gly Glu Thr Leu Ser Val Asn
            180                 185                 190
Asp Pro Pro Asp Val Leu Asp Arg Gln Lys Cys Leu Ala Ala Leu Ala
        195                 200                 205
Ser Leu Arg His Ala Lys Trp Phe Gln Ala Arg Ala Asn Gly Leu Lys
    210                 215                 220
Ser Cys Val Ile Val Ile Arg Val Leu Arg Asp Leu Cys Thr Arg Val
225                 230                 235                 240
Pro Thr Trp Gly Pro Leu Arg Gly Trp Pro Leu Glu Leu Leu Cys Glu
                245                 250                 255
Lys Ser Ile Val Thr Ala Asn Arg Pro Met Gly Ala Gly Glu Ala Leu
            260                 265                 270
Arg Arg Val Leu Glu Cys Leu Ala Ser Gly Ile Val Met Pro Asp Gly
        275                 280                 285
Ser Gly Ile Tyr Asp Pro Cys Glu Lys Glu Ala Thr Asp Ala Ile Gly
    290                 295                 300
His Leu Asp Arg Gln Gln Arg Glu Asp Ile Thr Gln Ser Ala Gln His
305                 310                 315                 320
Ala Leu Arg Leu Ala Ala Phe Gly Gln Leu His Lys Val Leu Gly Met
                325                 330                 335
Asp Pro Leu Pro Ser Lys Met Pro Lys Lys Pro Lys Asn Glu Asn Pro
            340                 345                 350
Val Asp Tyr Thr Val Gln Ile Pro Pro Ser Thr Thr Tyr Ala Ile Thr
        355                 360                 365
Pro Met Lys Arg Pro Met Glu Glu Asp Gly Glu Glu Lys Ser Pro Ser
    370                 375                 380
Lys Lys Lys Lys Lys Ile Gln Lys Lys Glu Glu Lys Ala Glu Pro Pro
385                 390                 395                 400
Gln Ala Met Asn Ala Leu Met Arg Leu Asn Gln Leu Lys Pro Gly Leu
                405                 410                 415
Gln Tyr Lys Leu Val Ser Gln Thr Gly Pro Val His Ala Pro Ile Phe
            420                 425                 430
Thr Met Ser Val Glu Val Asp Gly Asn Ser Phe Glu Ala Ser Gly Pro
        435                 440                 445
Ser Lys Lys Thr Ala Lys Leu His Val Ala Val Lys Val Leu Gln Asp
    450                 455                 460
Met Gly Leu Pro Thr Gly Ala Glu Gly Arg Asp Ser Ser Lys Gly Glu
465                 470                 475                 480
Asp Ser Ala Glu Glu Thr Glu Ala Lys Pro Ala Val Val Ala Pro Ala
                485                 490                 495

Pro Val Val Glu Ala Val Ser Thr Pro Ser Ala Ala Phe Pro Ser Asp
            500                 505                 510
Ala Thr Ala Glu Gln Gly Pro Ile Leu Thr Lys His Gly Lys Asn Pro
        515                 520                 525
Val Met Glu Leu Asn Glu Lys Arg Arg Gly Leu Lys Tyr Glu Leu Ile
    530                 535                 540
Ser Glu Thr Gly Gly Ser His Asp Lys Arg Phe Val Met Glu Val Glu
545                 550                 555                 560
Val Asp Gly Gln Lys Phe Gln Gly Ala Gly Ser Asn Lys Lys Val Ala
                565                 570                 575
Lys Ala Tyr Ala Ala Leu Ala Ala Leu Glu Lys Leu Phe Pro Asp Thr
            580                 585                 590
Pro Leu Ala Leu Asp Ala Asn Lys Lys Lys Arg Ala Pro Val Pro Val
        595                 600                 605
Arg Gly Gly
    610

<210> 15
<211> 702
<212> PRT
<213> Homo sapiens
<400> 15

<210> 16
<211> 440
<212> PRT
<213> Homo sapiens
<400> 16
Met Arg Pro Met Arg Ile Phe Val Asn Asp Asp Arg His Val Met Ala
1               5                   10                  15
Lys His Ser Ser Val Tyr Pro Thr Gln Glu Glu Leu Glu Ala Val Gln
            20                  25                  30
Asn Met Val Ser His Thr Glu Arg Ala Leu Lys Ala Val Ser Asp Trp
        35                  40                  45
Ile Asp Glu Gln Glu Lys Gly Ser Ser Glu Gln Ala Glu Ser Asp Asn
    50                  55                  60
Met Asp Val Pro Pro Glu Asp Asp Ser Lys Glu Gly Ala Gly Glu Gln
65                  70                  75                  80
Lys Thr Glu His Met Thr Arg Thr Leu Arg Gly Val Met Arg Val Gly
                85                  90                  95
Leu Val Ala Lys Gly Leu Leu Leu Lys Gly Asp Leu Asp Leu Glu Leu
            100                 105                 110
Val Leu Leu Cys Lys Glu Lys Pro Thr Thr Ala Leu Leu Asp Lys Val
        115                 120                 125
Ala Asp Asn Leu Ala Ile Gln Leu Ala Ala Val Thr Glu Asp Lys Tyr
    130                 135                 140
Glu Ile Leu Gln Ser Val Asp Asp Ala Ala Ile Val Ile Lys Asn Thr
145                 150                 155                 160
Lys Glu Pro Pro Leu Ser Leu Thr Ile His Leu Thr Ser Pro Val Val
                165                 170                 175

Arg Glu Glu Met Glu Lys Val Leu Ala Gly Glu Thr Leu Ser Val Asn
            180                 185                 190
Asp Pro Pro Asp Val Leu Asp Arg Gln Lys Cys Leu Ala Ala Leu Ala
        195                 200                 205
Ser Leu Arg His Ala Lys Trp Phe Gln Ala Arg Ala Asn Gly Leu Lys
    210                 215                 220
Ser Cys Val Ile Val Ile Arg Val Leu Arg Asp Leu Cys Thr Arg Val
225                 230                 235                 240
Pro Thr Trp Gly Pro Leu Arg Gly Trp Pro Leu Glu Leu Leu Cys Glu
                245                 250                 255
Lys Ser Ile Gly Thr Ala Asn Arg Pro Met Gly Ala Gly Glu Ala Leu
            260                 265                 270
Arg Arg Val Leu Glu Cys Leu Ala Ser Gly Ile Val Met Pro Asp Gly
        275                 280                 285
Ser Gly Ile Tyr Asp Pro Cys Glu Lys Glu Ala Thr Asp Ala Ile Gly
    290                 295                 300
His Leu Asp Arg Gln Gln Arg Glu Asp Ile Thr Gln Ser Ala Gln His
305                 310                 315                 320
Ala Leu Arg Leu Ala Ala Phe Gly Gln Leu His Lys Val Leu Gly Met
                325                 330                 335
Asp Pro Leu Pro Ser Lys Met Pro Lys Lys Pro Lys Asn Glu Asn Pro
            340                 345                 350
Val Asp Tyr Thr Val Gln Ile Pro Pro Ser Thr Thr Tyr Ala Ile Thr
        355                 360                 365
Pro Met Lys Arg Pro Met Glu Glu Asp Gly Glu Glu Lys Ser Pro Ser
    370                 375                 380
Lys Lys Lys Lys Lys Ile Gln Lys Lys Glu Glu Lys Ala Glu Pro Pro
385                 390                 395                 400
Gln Ala Met Asn Ala Leu Met Arg Leu Asn Gln Leu Lys Pro Gly Leu
                405                 410                 415
Gln Tyr Lys Leu Val Ser Gln Thr Gly Pro Val His Ala Pro Ile Phe
            420                 425                 430
Thr Met Ser Val Glu Val Asp Gly
        435                 440

<210> 17
<211> 345
<212> PRT
<213> Homo sapiens
<400> 17

<210> 18
<211> 313
<212> PRT
<213> Homo sapiens
<400> 18

<210> 19
<211> 741
<212> PRT
<213> Homo sapiens
<400> 19
Met Asp Ile Glu Asp Glu Glu Asn Met Ser Ser Ser Ser Thr Asp Val
1               5                   10                  15

Lys Glu Asn Arg Asn Leu Asp Asn Val Ser Pro Lys Asp Gly Ser Thr
                20                  25                  30
Pro Gly Pro Gly Glu Gly Ser Gln Leu Ser Asn Gly Gly Gly Gly Gly
            35                  40                  45
Pro Gly Arg Lys Arg Pro Leu Glu Glu Gly Ser Asn Gly His Ser Lys
        50                  55                  60
Tyr Arg Leu Lys Lys Arg Arg Lys Thr Pro Gly Pro Val Leu Pro Lys
65                  70                  75                  80
Asn Ala Leu Met Gln Leu Asn Glu Ile Lys Pro Gly Leu Gln Tyr Thr
                85                  90                  95
Leu Leu Ser Gln Thr Gly Pro Val His Ala Pro Leu Phe Val Met Ser
                100                 105                 110
Val Glu Val Asn Gly Gln Val Phe Glu Gly Ser Gly Pro Thr Lys Lys
            115                 120                 125
Lys Ala Lys Leu His Ala Ala Glu Lys Ala Leu Arg Ser Phe Val Gln
        130                 135                 140
Phe Pro Asn Ala Ser Glu Ala His Leu Ala Met Gly Arg Thr Leu Ser
145                 150                 155                 160
Val Asn Thr Asp Phe Thr Ser Asp Gln Ala Asp Phe Pro Asp Thr Leu
                165                 170                 175
Phe Asn Gly Phe Glu Thr Pro Asp Lys Ala Glu Pro Pro Phe Tyr Val
                180                 185                 190
Gly Ser Asn Gly Asp Asp Ser Phe Ser Ser Ser Gly Asp Leu Ser Leu
            195                 200                 205
Ser Ala Ser Pro Val Pro Ala Ser Leu Ala Gln Pro Pro Leu Pro Val
    210                 215                 220
Leu Pro Pro Phe Pro Pro Pro Ser Gly Lys Asn Pro Val Met Ile Leu
225                 230                 235                 240
Asn Glu Leu Arg Pro Gly Leu Lys Tyr Asp Phe Leu Ser Glu Ser Gly
                245                 250                 255
Glu Ser His Ala Lys Ser Phe Val Met Ser Val Val Val Asp Gly Gln
                260                 265                 270
Phe Phe Glu Gly Ser Gly Arg Asn Lys Lys Leu Ala Lys Ala Arg Ala
            275                 280                 285
Ala Gln Ser Ala Leu Ala Ala Ile Phe Asn Leu His Leu Asp Gln Thr
    290                 295                 300
Pro Ser Arg Gln Pro Ile Pro Ser Glu Gly Leu Gln Leu His Leu Pro
305                 310                 315                 320
Gln Val Leu Ala Asp Ala Val Ser Arg Leu Val Leu Gly Lys Phe Gly
                325                 330                 335
Asp Leu Thr Asp Asn Phe Ser Ser Pro His Ala Arg Arg Lys Val Leu
                340                 345                 350
Ala Gly Val Val Met Thr Thr Gly Thr Asp Val Lys Asp Ala Lys Val
            355                 360                 365
Ile Ser Val Ser Thr Gly Thr Lys Cys Ile Asn Gly Glu Tyr Met Ser
    370                 375                 380

Asp Arg Gly Leu Ala Leu Asn Asp Cys His Ala Glu Ile Ile Ser Arg
385                 390                 395                 400
Arg Ser Leu Leu Arg Phe Leu Tyr Thr Gln Leu Glu Leu Tyr Leu Asn
                405                 410                 415
Asn Lys Asp Asp Gln Lys Arg Ser Ile Phe Gln Lys Ser Glu Arg Gly
            420                 425                 430
Gly Phe Arg Leu Lys Glu Asn Val Gln Phe His Leu Tyr Ile Ser Thr
        435                 440                 445
Ser Pro Cys Gly Asp Ala Arg Ile Phe Ser Pro His Glu Pro Ile Leu
    450                 455                 460
Glu Gly Ser Arg Ser Tyr Thr Gln Ala Gly Val Gln Trp Cys Asn His
465                 470                 475                 480
Gly Ser Leu Gln Pro Arg Pro Pro Gly Leu Leu Ser Asp Pro Ser Thr
                485                 490                 495
Ser Thr Phe Gln Gly Ala Gly Thr Thr Glu Pro Ala Asp Arg His Pro
            500                 505                 510
Asn Arg Lys Ala Arg Gly Gln Leu Arg Thr Lys Ile Glu Ser Gly Glu
        515                 520                 525
Gly Thr Ile Pro Val Arg Ser Asn Ala Ser Ile Gln Thr Trp Asp Gly
    530                 535                 540
Val Leu Gln Gly Glu Arg Leu Leu Thr Met Ser Cys Ser Asp Lys Ile
545                 550                 555                 560
Ala Arg Trp Asn Val Val Gly Ile Gln Gly Ser Leu Leu Ser Ile Phe
                565                 570                 575
Val Glu Pro Ile Tyr Phe Ser Ser Ile Ile Leu Gly Ser Leu Tyr His
            580                 585                 590
Gly Asp His Leu Ser Arg Ala Met Tyr Gln Arg Ile Ser Asn Ile Glu
        595                 600                 605
Asp Leu Pro Pro Leu Tyr Thr Leu Asn Lys Pro Leu Leu Ser Gly Ile
    610                 615                 620
Ser Asn Ala Glu Ala Arg Gln Pro Gly Lys Ala Pro Asn Phe Ser Val
625                 630                 635                 640
Asn Trp Thr Val Gly Asp Ser Ala Ile Glu Val Ile Asn Ala Thr Thr
                645                 650                 655
Gly Lys Asp Glu Leu Gly Arg Ala Ser Arg Leu Cys Lys His Ala Leu
            660                 665                 670
Tyr Cys Arg Trp Met Arg Val His Gly Lys Val Pro Ser His Leu Leu
        675                 680                 685
Arg Ser Lys Ile Thr Lys Pro Asn Val Tyr His Glu Ser Lys Leu Ala
    690                 695                 700
Ala Lys Glu Tyr Gln Ala Ala Lys Ala Arg Leu Phe Thr Ala Phe Ile
705                 710                 715                 720
Lys Ala Gly Leu Gly Ala Trp Val Glu Lys Pro Thr Glu Gln Asp Gln
                725                 730                 735
Phe Ser Leu Thr Pro
            740

<210> 20
<211> 1225
<212> PRT
<213> Homo sapiens
<400> 20
Met Asn Pro Arg Gln Gly Tyr Ser Leu Ser Gly Tyr Tyr Thr His Pro
1               5                   10                  15
Phe Gln Gly Tyr Glu His Arg Gln Leu Arg Tyr Gln Gln Pro Gly Pro
            20                  25                  30
Gly Ser Ser Pro Ser Ser Phe Leu Leu Lys Gln Ile Glu Phe Leu Lys
        35                  40                  45
Gly Gln Leu Pro Glu Ala Pro Val Ile Gly Lys Gln Thr Pro Ser Leu
    50                  55                  60
Pro Pro Ser Leu Pro Gly Leu Arg Pro Arg Phe Pro Val Leu Leu Ala
65                  70                  75                  80
Ser Ser Thr Arg Gly Arg Gln Val Asp Ile Arg Gly Val Pro Arg Gly
                85                  90                  95
Val His Leu Gly Ser Gln Gly Leu Gln Arg Gly Phe Gln His Pro Ser
            100                 105                 110
Pro Arg Gly Arg Ser Leu Pro Gln Arg Gly Val Asp Cys Leu Ser Ser
        115                 120                 125
His Phe Gln Glu Leu Ser Ile Tyr Gln Asp Gln Glu Gln Arg Ile Leu
    130                 135                 140
Lys Phe Leu Glu Glu Leu Gly Glu Gly Lys Ala Thr Thr Ala His Asp
145                 150                 155                 160
Leu Ser Gly Lys Leu Gly Thr Pro Lys Lys Glu Ile Asn Arg Val Leu
                165                 170                 175
Tyr Ser Leu Ala Lys Lys Gly Lys Leu Gln Lys Glu Ala Gly Thr Pro
            180                 185                 190
Pro Leu Trp Lys Ile Ala Val Ser Thr Gln Ala Trp Asn Gln His Ser
        195                 200                 205
Gly Val Val Arg Pro Asp Gly His Ser Gln Gly Ala Pro Asn Ser Asp
    210                 215                 220
Pro Ser Leu Glu Pro Glu Asp Arg Asn Ser Thr Ser Val Ser Glu Asp
225                 230                 235                 240
Leu Leu Glu Pro Phe Ile Ala Val Ser Ala Gln Ala Trp Asn Gln His
                245                 250                 255
Ser Gly Val Val Arg Pro Asp Ser His Ser Gln Gly Ser Pro Asn Ser
            260                 265                 270
Asp Pro Gly Leu Glu Pro Glu Asp Ser Asn Ser Thr Ser Ala Leu Glu
        275                 280                 285
Asp Pro Leu Glu Phe Leu Asp Met Ala Glu Ile Lys Glu Lys Ile Cys
    290                 295                 300
Asp Tyr Leu Phe Asn Val Ser Asp Ser Ser Ala Leu Asn Leu Ala Lys
305                 310                 315                 320

Asn Ile Gly Leu Thr Lys Ala Arg Asp Ile Asn Ala Val Leu Ile Asp
                325                 330                 335
Met Glu Arg Gln Gly Asp Val Tyr Arg Gln Gly Thr Thr Pro Pro Ile
            340                 345                 350
Trp His Leu Thr Asp Lys Lys Arg Glu Arg Met Gln Ile Lys Arg Asn
        355                 360                 365
Thr Asn Ser Val Pro Glu Thr Ala Pro Ala Ala Ile Pro Glu Thr Arg
    370                 375                 380
Arg Asn Ala Glu Phe Leu Thr Cys Asn Ile Pro Thr Ser Asn Ala Ser
385                 390                 395                 400
Asn Asn Met Val Thr Thr Glu Lys Val Glu Asn Gly Gln Glu Pro Val
                405                 410                 415
Lys Leu Glu Asn Arg Gln Glu Ala Arg Pro Glu Pro Ala Arg Leu Lys
            420                 425                 430
Pro Pro Val His Tyr Asn Gly Pro Ser Lys Ala Gly Tyr Val Asp Phe
        435                 440                 445
Glu Asn Gly Gln Trp Ala Thr Asp Asp Ile Pro Asp Asp Leu Asn Ser
    450                 455                 460
Ile Arg Ala Ala Pro Gly Glu Phe Arg Ala Ile Met Glu Met Pro Ser
465                 470                 475                 480
Phe Tyr Ser His Gly Leu Pro Arg Cys Ser Pro Tyr Lys Lys Leu Thr
                485                 490                 495
Glu Cys Gln Leu Lys Asn Pro Ile Ser Gly Leu Leu Glu Tyr Ala Gln
            500                 505                 510
Phe Ala Ser Gln Thr Cys Glu Phe Asn Met Ile Glu Gln Ser Gly Pro
        515                 520                 525
Pro His Glu Pro Arg Phe Lys Phe Gln Val Val Ile Asn Gly Arg Glu
    530                 535                 540
Phe Pro Pro Ala Glu Ala Gly Ser Lys Lys Val Ala Lys Gln Asp Ala
545                 550                 555                 560
Ala Met Lys Ala Met Thr Ile Leu Leu Glu Glu Ala Lys Ala Lys Asp
                565                 570                 575
Ser Gly Lys Ser Glu Glu Ser Ser His Tyr Ser Thr Glu Lys Glu Ser
            580                 585                 590
Glu Lys Thr Ala Glu Ser Gln Thr Pro Thr Pro Ser Ala Thr Ser Phe
        595                 600                 605
Phe Ser Gly Lys Ser Pro Val Thr Thr Leu Leu Glu Cys Met His Lys
    610                 615                 620
Leu Gly Asn Ser Cys Glu Phe Arg Leu Leu Ser Lys Glu Gly Pro Ala
625                 630                 635                 640
His Glu Pro Lys Phe Gln Tyr Cys Val Ala Val Gly Ala Gln Thr Phe
                645                 650                 655
Pro Ser Val Ser Ala Pro Ser Lys Lys Val Ala Lys Gln Met Ala Ala
            660                 665                 670
Glu Glu Ala Met Lys Ala Leu His Gly Glu Ala Thr Asn Ser Met Ala
        675                 680                 685

Ser Asp Asn Gln Pro Glu Gly Met Ile Ser Glu Ser Leu Asp Asn Leu
    690                 695                 700
Glu Ser Met Met Pro Asn Lys Val Arg Lys Ile Gly Glu Leu Val Arg
705                 710                 715                 720
Tyr Leu Asn Thr Asn Pro Val Gly Gly Leu Leu Glu Tyr Ala Arg Ser
                725                 730                 735
His Gly Phe Ala Ala Glu Phe Lys Leu Val Asp Gln Ser Gly Pro Pro
            740                 745                 750
His Glu Pro Lys Phe Val Tyr Gln Ala Lys Val Gly Gly Arg Trp Phe
        755                 760                 765
Pro Ala Val Cys Ala His Ser Lys Lys Gln Gly Lys Gln Glu Ala Ala
    770                 775                 780
Asp Ala Ala Leu Arg Val Leu Ile Gly Glu Asn Glu Lys Ala Glu Arg
785                 790                 795                 800
Met Gly Phe Thr Glu Val Thr Pro Val Thr Gly Ala Ser Leu Arg Arg
                805                 810                 815
Thr Met Leu Leu Leu Ser Arg Ser Pro Glu Ala Gln Pro Lys Thr Leu
            820                 825                 830
Pro Leu Thr Gly Ser Thr Phe His Asp Gln Ile Ala Met Leu Ser His
        835                 840                 845
Arg Cys Phe Asn Thr Leu Thr Asn Ser Phe Gln Pro Ser Leu Leu Gly
    850                 855                 860
Arg Lys Ile Leu Ala Ala Ile Ile Met Lys Lys Asp Ser Glu Asp Met
865                 870                 875                 880
Gly Val Val Val Ser Leu Gly Thr Gly Asn Arg Cys Val Lys Gly Asp
                885                 890                 895
Ser Leu Ser Leu Lys Gly Glu Thr Val Asn Asp Cys His Ala Glu Ile
            900                 905                 910
Ile Ser Arg Arg Gly Phe Ile Arg Phe Leu Tyr Ser Glu Leu Met Lys
        915                 920                 925
Tyr Asn Ser Gln Thr Ala Lys Asp Ser Ile Phe Glu Pro Ala Lys Gly
    930                 935                 940
Gly Glu Lys Leu Gln Ile Lys Lys Thr Val Ser Phe His Leu Tyr Ile
945                 950                 955                 960
Ser Thr Ala Pro Cys Gly Asp Gly Ala Leu Phe Asp Lys Ser Cys Ser
                965                 970                 975
Asp Arg Ala Met Glu Ser Thr Glu Ser Arg His Tyr Pro Val Phe Glu
            980                 985                 990
Asn Pro Lys Gln Gly Lys Leu Arg Thr Lys Val Glu Asn Gly Glu Gly
        995                 1000                1005
Thr Ile Pro Val Glu Ser Ser Asp Ile Val Pro Thr Trp Asp Gly Ile
    1010                1015                1020
Arg Leu Gly Glu Arg Leu Arg Thr Met Ser Cys Ser Asp Lys Ile Leu
1025                1030                1035                1040
Arg Trp Asn Val Leu Gly Leu Gln Gly Ala Leu Leu Thr His Phe Leu
                1045                1050                1055

Gln Pro Ile Tyr Leu Lys Ser Val Thr Leu Gly Tyr Leu Phe Ser Gln
            1060                1065                1070
Gly His Leu Thr Arg Ala Ile Cys Cys Arg Val Thr Arg Asp Gly Ser
        1075                1080                1085
Ala Phe Glu Asp Gly Leu Arg His Pro Phe Ile Val Asn His Pro Lys
    1090                1095                1100
Val Gly Arg Val Ser Ile Tyr Asp Ser Lys Arg Gln Ser Gly Lys Thr
1105                1110                1115                1120
Lys Glu Thr Ser Val Asn Trp Cys Leu Ala Asp Gly Tyr Asp Leu Glu
                1125                1130                1135
Ile Leu Asp Gly Thr Arg Gly Thr Val Asp Gly Pro Arg Asn Glu Leu
            1140                1145                1150
Ser Arg Val Ser Lys Lys Asn Ile Phe Leu Leu Phe Lys Lys Leu Cys
        1155                1160                1165
Ser Phe Arg Tyr Arg Arg Asp Leu Leu Arg Leu Ser Tyr Gly Glu Ala
    1170                1175                1180
Lys Lys Ala Ala Arg Asp Tyr Glu Thr Ala Lys Asn Tyr Phe Lys Lys
1185                1190                1195                1200
Gly Leu Lys Asp Met Gly Tyr Gly Asn Trp Ile Ser Lys Pro Gln Glu
                1205                1210                1215
Glu Lys Asn Phe Tyr Leu Cys Pro Val
            1220                1225

<210> 21
<211> 702
<212> PRT
<213> Homo sapiens
<400> 21
Met Arg Pro Met Arg Ile Phe Val Asn Asp Asp Arg His Val Met Ala
1               5                   10                  15
Lys His Ser Ser Val Tyr Pro Thr Gln Glu Glu Leu Glu Ala Val Gln
            20                  25                  30
Asn Met Val Ser His Thr Glu Arg Ala Leu Lys Ala Val Ser Asp Trp
        35                  40                  45
Ile Asp Glu Gln Glu Lys Gly Ser Ser Glu Gln Ala Glu Ser Asp Asn
    50                  55                  60
Met Asp Val Pro Pro Glu Asp Asp Ser Lys Glu Gly Ala Gly Glu Gln
65                  70                  75                  80
Lys Thr Glu His Met Thr Arg Thr Leu Arg Gly Val Met Arg Val Gly
                85                  90                  95
Leu Val Ala Lys Gly Leu Leu Leu Lys Gly Asp Leu Asp Leu Glu Leu
            100                 105                 110
Val Leu Leu Cys Lys Glu Lys Pro Thr Thr Ala Leu Leu Asp Lys Val
        115                 120                 125
Ala Asp Asn Leu Ala Ile Gln Leu Ala Ala Val Thr Glu Asp Lys Tyr
    130                 135                 140

Glu Ile Leu Gln Ser Val Asp Asp Ala Ala Ile Val Ile Lys Asn Thr
145                 150                 155                 160
Lys Glu Pro Pro Leu Ser Leu Thr Ile His Leu Thr Ser Pro Val Val
                165                 170                 175
Arg Glu Glu Met Glu Lys Val Leu Ala Gly Glu Thr Leu Ser Val Asn
            180                 185                 190
Asp Pro Pro Asp Val Leu Asp Arg Gln Lys Cys Leu Ala Ala Leu Ala
        195                 200                 205
Ser Leu Arg His Ala Lys Trp Phe Gln Ala Arg Ala Asn Gly Leu Lys
    210                 215                 220
Ser Cys Val Ile Val Ile Arg Val Leu Arg Asp Leu Cys Thr Arg Val
225                 230                 235                 240
Pro Thr Trp Gly Pro Leu Arg Gly Trp Pro Leu Glu Leu Leu Cys Glu
                245                 250                 255
Lys Ser Ile Gly Thr Ala Asn Arg Pro Met Gly Ala Gly Glu Ala Leu
            260                 265                 270
Arg Arg Val Leu Glu Cys Leu Ala Ser Gly Ile Val Met Pro Asp Gly
        275                 280                 285
Ser Gly Ile Tyr Asp Pro Cys Glu Lys Glu Ala Thr Asp Ala Ile Gly
    290                 295                 300
His Leu Asp Arg Gln Gln Arg Glu Asp Ile Thr Gln Ser Ala Gln His
305                 310                 315                 320
Ala Leu Arg Leu Ala Ala Phe Gly Gln Leu His Lys Val Leu Gly Met
                325                 330                 335
Asp Pro Leu Pro Ser Lys Met Pro Lys Lys Pro Lys Asn Glu Asn Pro
            340                 345                 350
Val Asp Tyr Thr Val Gln Ile Pro Pro Ser Thr Thr Tyr Ala Ile Thr
        355                 360                 365
Pro Met Lys Arg Pro Met Glu Glu Asp Gly Glu Glu Lys Ser Pro Ser
    370                 375                 380
Lys Lys Lys Lys Lys Ile Gln Lys Lys Glu Glu Lys Ala Glu Pro Pro
385                 390                 395                 400
Gln Ala Met Asn Ala Leu Met Arg Leu Asn Gln Leu Lys Pro Gly Leu
                405                 410                 415
Gln Tyr Lys Leu Val Ser Gln Thr Gly Pro Val His Ala Pro Ile Phe
            420                 425                 430
Thr Met Ser Val Glu Val Asp Gly Asn Ser Phe Glu Ala Ser Gly Pro
        435                 440                 445
Ser Lys Lys Thr Ala Lys Leu His Val Ala Val Lys Val Leu Gln Asp
    450                 455                 460
Met Gly Leu Pro Thr Gly Ala Glu Gly Arg Asp Ser Ser Lys Gly Glu
465                 470                 475                 480
Asp Ser Ala Glu Glu Thr Glu Ala Lys Pro Ala Val Val Ala Pro Ala
                485                 490                 495
Pro Val Val Glu Ala Val Ser Thr Pro Ser Ala Ala Phe Pro Ser Asp
            500                 505                 510

Ala Thr Ala Glu Gln Gly Pro Ile Leu Thr Lys His Gly Lys Asn Pro
        515                 520                 525
Val Met Glu Leu Asn Glu Lys Arg Arg Gly Leu Lys Tyr Glu Leu Ile
    530                 535                 540
Ser Glu Thr Gly Gly Ser His Asp Lys Arg Phe Val Met Glu Val Glu
545                 550                 555                 560
Val Asp Gly Gln Lys Phe Gln Gly Ala Gly Ser Asn Lys Lys Val Ala
                565                 570                 575
Lys Ala Tyr Ala Ala Leu Ala Ala Leu Glu Lys Leu Phe Pro Asp Thr
            580                 585                 590
Pro Leu Ala Leu Asp Ala Asn Lys Lys Lys Arg Ala Pro Val Pro Val
        595                 600                 605
Arg Gly Gly Pro Lys Phe Ala Ala Lys Pro His Asn Pro Gly Phe Gly
    610                 615                 620
Met Gly Gly Pro Met His Asn Glu Val Pro Pro Pro Pro Asn Leu Arg
625                 630                 635                 640
Gly Arg Gly Arg Gly Gly Ser Ile Arg Gly Arg Gly Arg Gly Arg Gly
                645                 650                 655
Phe Gly Gly Ala Asn His Gly Gly Tyr Met Asn Ala Gly Ala Gly Tyr
            660                 665                 670
Gly Ser Tyr Gly Tyr Gly Gly Asn Ser Ala Thr Ala Gly Tyr Ser Asp
        675                 680                 685
Phe Phe Thr Asp Cys Tyr Gly Tyr His Asp Phe Gly Ser Ser
    690                 695                 700

<210> 22
<211> 764
<212> PRT
<213> Homo sapiens
<400> 22
Met Arg Pro Met Arg Ile Phe Val Asn Asp Asp Arg His Val Met Ala
  1               5                  10                  15
Lys His Ser Ser Val Tyr Pro Thr Gln Glu Glu Leu Glu Ala Val Gln
             20                  25                  30
Asn Met Val Ser His Thr Glu Arg Ala Leu Lys Ala Val Ser Asp Trp
         35                  40                  45
Ile Asp Glu Gln Glu Lys Gly Ser Ser Glu Gln Ala Glu Ser Asp Asn
     50                  55                  60
Met Asp Val Pro Pro Glu Asp Asp Ser Lys Glu Gly Ala Gly Glu Gln
 65                  70                  75                  80
Lys Thr Glu His Met Thr Arg Thr Leu Arg Gly Val Met Arg Val Gly
                 85                  90                  95
Leu Val Ala Lys Gly Leu Leu Leu Lys Gly Asp Leu Asp Leu Glu Leu
            100                 105                 110
Val Leu Leu Cys Lys Glu Lys Pro Thr Thr Ala Leu Leu Asp Lys Val
        115                 120                 125

Ala Asp Asn Leu Ala Ile Gln Leu Ala Ala Val Thr Glu Asp Lys Tyr
    130                 135                 140
Glu Ile Leu Gln Ser Val Asp Asp Ala Ala Ile Val Ile Lys Asn Thr
145                 150                 155                 160
Lys Glu Pro Pro Leu Ser Leu Thr Ile His Leu Thr Ser Pro Val Val
                165                 170                 175
Arg Glu Glu Met Glu Lys Val Leu Ala Gly Glu Thr Leu Ser Val Asn
            180                 185                 190
Asp Pro Pro Asp Val Leu Asp Arg Gln Lys Cys Leu Ala Ala Leu Ala
        195                 200                 205
Ser Leu Arg His Ala Lys Trp Phe Gln Ala Arg Ala Asn Gly Leu Lys
    210                 215                 220
Ser Cys Val Ile Val Ile Arg Val Leu Arg Asp Leu Cys Thr Arg Val
225                 230                 235                 240
Pro Thr Trp Gly Pro Leu Arg Gly Trp Pro Leu Glu Leu Leu Cys Glu
                245                 250                 255
Lys Ser Ile Gly Thr Ala Asn Arg Pro Met Gly Ala Gly Glu Ala Leu
            260                 265                 270
Arg Arg Val Leu Glu Cys Leu Ala Ser Gly Ile Val Met Pro Asp Gly
        275                 280                 285
Ser Gly Ile Tyr Asp Pro Cys Glu Lys Glu Ala Thr Asp Ala Ile Gly
    290                 295                 300
His Leu Asp Arg Gln Gln Arg Glu Asp Ile Thr Gln Ser Ala Gln His
305                 310                 315                 320
Ala Leu Arg Leu Ala Ala Phe Gly Gln Leu His Lys Val Leu Gly Met
                325                 330                 335
Asp Pro Leu Pro Ser Lys Met Pro Lys Lys Pro Lys Asn Glu Asn Pro
            340                 345                 350
Val Asp Tyr Thr Val Gln Ile Pro Pro Ser Thr Thr Tyr Ala Ile Thr
        355                 360                 365
Pro Met Lys Arg Pro Met Glu Glu Asp Gly Glu Glu Lys Ser Pro Ser
    370                 375                 380
Lys Lys Lys Lys Lys Ile Gln Lys Lys Glu Glu Lys Ala Glu Pro Pro
385                 390                 395                 400
Gln Ala Met Asn Ala Leu Met Arg Leu Asn Gln Leu Lys Pro Gly Leu
                405                 410                 415
Gln Tyr Lys Leu Val Ser Gln Thr Gly Pro Val His Ala Pro Ile Phe
            420                 425                 430
Thr Met Ser Val Glu Val Asp Gly Asn Ser Phe Glu Ala Ser Gly Pro
        435                 440                 445
Ser Lys Lys Thr Ala Lys Leu His Val Ala Val Lys Val Leu Gln Asp
    450                 455                 460
Met Gly Leu Pro Thr Gly Ala Glu Gly Arg Asp Ser Ser Lys Gly Glu
465                 470                 475                 480
Asp Ser Ala Glu Glu Thr Glu Ala Lys Pro Ala Val Val Ala Pro Ala
                485                 490                 495

Pro Val Val Glu Ala Val Ser Thr Pro Ser Ala Ala Phe Pro Ser Asp
            500                 505                 510
Ala Thr Ala Glu Gln Gly Pro Ile Leu Thr Lys His Gly Lys Asn Pro
        515                 520                 525
Val Met Glu Leu Asn Glu Lys Arg Arg Gly Leu Lys Tyr Glu Leu Ile
    530                 535                 540
Ser Glu Thr Gly Gly Ser His Asp Lys Arg Phe Val Met Glu Val Glu
545                 550                 555                 560
Val Asp Gly Gln Lys Phe Gln Gly Ala Gly Ser Asn Lys Lys Val Ala
                565                 570                 575
Lys Ala Tyr Ala Ala Leu Ala Ala Leu Glu Lys Leu Phe Pro Asp Thr
            580                 585                 590
Pro Leu Ala Leu Asp Ala Asn Lys Lys Lys Arg Ala Pro Val Pro Val
        595                 600                 605
Arg Gly Gly Pro Lys Phe Ala Ala Lys Pro His Asn Pro Gly Phe Gly
    610                 615                 620
Met Gly Gly Pro Met His Asn Glu Val Pro Pro Pro Pro Asn Leu Arg
625                 630                 635                 640
Gly Arg Gly Arg Gly Gly Ser Ile Arg Gly Arg Gly Arg Gly Arg Gly
                645                 650                 655
Phe Gly Gly Ala Asn His Gly Gly Tyr Met Asn Ala Gly Ala Gly Tyr
            660                 665                 670
Gly Ser Tyr Gly Tyr Gly Gly Asn Ser Ala Thr Ala Gly Tyr Ser Gln
        675                 680                 685
Phe Ser Arg Pro Pro Pro Pro Ser Arg Pro Arg Cys Cys Val Val Arg
    690                 695                 700
Cys Ser Gly Ser Pro Cys Gly Pro Ser Cys Asp Pro Tyr Leu Ala Val
705                 710                 715                 720
Phe Gly Thr Pro Cys Leu Gln Trp Phe Val Ser Cys His Tyr Asn Phe
                725                 730                 735
Val Trp Val Glu Phe Leu Ser Phe Cys Ser Ser Val Ser Leu Cys Leu
            740                 745                 750
Phe Thr Leu Arg Val Ser Gly Asn Ser Val Cys Leu
        755                 760

<210> 23
<211> 591
<212> PRT
<213> Homo sapiens
<400> 23
Gly His Leu Asp Arg Gln Gln Arg Glu Asp Ile Thr Gln Ser Ala Gln
1               5                   10                  15
His Ala Leu Arg Leu Ala Ala Phe Gly Gln Leu His Lys Val Leu Gly
            20                  25                  30
Met Asp Pro Leu Pro Ser Lys Met Pro Lys Lys Pro Lys Asn Glu Asn
        35                  40                  45

Pro Val Asp Tyr Thr Val Gln Ile Pro Pro Ser Thr Thr Tyr Ala Ile
    50                  55                  60
Thr Pro Met Lys Arg Pro Met Glu Glu Asp Gly Glu Glu Lys Ser Pro
65                  70                  75                  80
Ser Lys Lys Lys Lys Lys Ile Gln Lys Lys Glu Glu Lys Ala Glu Pro
                85                  90                  95
Pro Gln Ala Met Asn Ala Leu Met Arg Leu Asn Gln Leu Lys Pro Gly
            100                 105                 110
Leu Gln Tyr Lys Leu Val Ser Gln Thr Gly Pro Val His Ala Pro Ile
        115                 120                 125
Phe Thr Met Ser Val Glu Val Asp Gly Asn Ser Phe Glu Ala Ser Gly
    130                 135                 140
Pro Ser Lys Lys Thr Ala Lys Leu His Val Ala Val Lys Val Leu Gln
145                 150                 155                 160
Asp Met Gly Leu Pro Thr Gly Ala Glu Gly Arg Asp Ser Ser Lys Gly
                165                 170                 175
Glu Asp Ser Ala Glu Glu Thr Glu Ala Lys Pro Ala Val Val Ala Pro
            180                 185                 190
Ala Pro Val Val Glu Ala Val Ser Thr Pro Ser Ala Ala Phe Pro Ser
        195                 200                 205
Asp Ala Thr Ala Glu Gln Gly Pro Ile Leu Thr Lys His Gly Lys Asn
    210                 215                 220
Pro Val Met Glu Leu Asn Glu Lys Arg Arg Gly Leu Lys Tyr Glu Leu
225                 230                 235                 240
Ile Ser Glu Thr Gly Gly Ser His Asp Lys Arg Phe Val Met Glu Val
                245                 250                 255
Glu Val Asp Gly Gln Lys Phe Gln Gly Ala Gly Ser Asn Lys Lys Val
            260                 265                 270
Ala Lys Ala Tyr Ala Ala Leu Ala Ala Leu Glu Lys Leu Phe Pro Asp
        275                 280                 285
Thr Pro Leu Ala Leu Asp Ala Asn Lys Lys Lys Arg Ala Pro Val Pro
    290                 295                 300
Val Arg Gly Gly Pro Lys Phe Ala Ala Lys Pro His Asn Pro Gly Phe
305                 310                 315                 320
Gly Met Gly Gly Pro Met His Asn Glu Val Pro Pro Pro Pro Asn Leu
                325                 330                 335
Arg Gly Arg Gly Arg Gly Gly Ser Ile Arg Gly Arg Gly Arg Gly Arg
            340                 345                 350
Gly Phe Gly Gly Ala Asn His Gly Gly Tyr Met Asn Ala Gly Ala Gly
        355                 360                 365
Tyr Gly Ser Tyr Gly Tyr Gly Gly Asn Ser Ala Thr Ala Gly Tyr Ser
    370                 375                 380
Gln Phe Tyr Ser Asn Gly Gly His Ser Gly Asn Ala Ser Gly Gly Gly
385                 390                 395                 400
Gly Gly Gly Gly Gly Gly Ser Ser Gly Tyr Gly Ser Tyr Tyr Gln Gly
                405                 410                 415

Asp Asn Tyr Asn Ser Pro Val Pro Pro Lys His Ala Gly Lys Lys Gln
            420                 425                 430
Pro His Gly Gly Gln Gln Lys Pro Ser Tyr Gly Ser Gly Tyr Gln Ser
        435                 440                 445
His Gln Gly Gln Gln Gln Ser Tyr Asn Gln Ser Leu Tyr Ser Asn Tyr
    450                 455                 460
Gly Pro Pro Gln Gly Lys Gln Lys Gly Tyr Asn His Gly Gln Gly Ser
465                 470                 475                 480
Tyr Ser Tyr Ser Asn Ser Tyr Asn Ser Pro Gly Gly Gly Gly Gly Ser
                485                 490                 495
Asp Tyr Asn Tyr Glu Ser Lys Phe Asn Tyr Ser Gly Ser Gly Gly Arg
            500                 505                 510
Ser Gly Gly Asn Ser Tyr Gly Ser Gly Gly Ala Ser Tyr Asn Pro Gly
        515                 520                 525
Ser His Gly Gly Tyr Gly Gly Gly Ser Gly Gly Gly Ser Ser Tyr Gln
    530                 535                 540
Gly Lys Gln Gly Gly Tyr Ser Gln Ser Asn Tyr Asn Ser Pro Gly Ser
545                 550                 555                 560
Gly Gln Asn Tyr Ser Gly Pro Pro Ser Ser Tyr Gln Ser Ser Gln Gly
                565                 570                 575
Gly Tyr Gly Arg Asn Ala Asp His Ser Met Asn Tyr Gln Tyr Arg
            580                 585                 590

<210> 24
<211> 514
<212> PRT
<213> Homo sapiens
<400> 24
Thr Leu Ser Val Asn Asp Pro Pro Asp Val Leu Asp Arg Gln Lys Cys
1               5                   10                  15
Leu Ala Ala Leu Ala Ser Leu Arg His Ala Lys Trp Phe Gln Ala Arg
            20                  25                  30
Ala Asn Gly Leu Lys Ser Cys Val Ile Val Ile Arg Val Leu Arg Asp
        35                  40                  45
Leu Cys Thr Arg Val Pro Thr Gly Pro Leu Arg Gly Trp Pro Leu Glu
    50                  55                  60
Leu Leu Cys Glu Lys Ser Ile Gly Thr Ala Asn Arg Pro Met Gly Ala
65                  70                  75                  80
Gly Glu Ala Leu Arg Arg Val Leu Glu Cys Leu Ala Ser Gly Ile Val
                85                  90                  95
Met Pro Asp Gly Ser Gly Ile Tyr Asp Pro Cys Glu Lys Glu Ala Thr
            100                 105                 110
Asp Ala Ile Gly His Leu Asp Arg Gln Gln Arg Glu Asp Ile Thr Gln
        115                 120                 125
Ser Ala Gln His Ala Leu Arg Leu Ala Ala Phe Gly Gln Leu His Lys
    130                 135                 140

Val Leu Gly Met Asp Pro Leu Pro Ser Lys Met Pro Lys Lys Pro Lys
145                 150                 155                 160
Asn Glu Asn Pro Val Asp Tyr Thr Val Gln Ile Pro Pro Ser Thr Thr
                165                 170                 175
Tyr Ala Ile Thr Pro Met Lys Arg Pro Met Glu Glu Asp Gly Glu Glu
            180                 185                 190
Lys Ser Pro Ser Lys Lys Lys Lys Lys Ile Gln Lys Lys Glu Glu Lys
        195                 200                 205
Ala Glu Pro Pro Gln Ala Met Asn Ala Leu Met Arg Leu Asn Gln Leu
    210                 215                 220
Lys Pro Gly Leu Gln Tyr Lys Leu Val Ser Gln Thr Gly Pro Val His
225                 230                 235                 240
Ala Pro Ile Phe Thr Met Ser Val Glu Val Asp Gly Asn Ser Phe Glu
                245                 250                 255
Ala Ser Gly Pro Ser Lys Lys Thr Ala Lys Leu His Val Ala Val Lys
            260                 265                 270
Val Leu Gln Asp Met Gly Leu Pro Thr Gly Ala Glu Gly Arg Asp Ser
        275                 280                 285
Ser Lys Gly Glu Asp Ser Ala Glu Glu Thr Glu Ala Lys Pro Ala Val
    290                 295                 300
Val Ala Pro Ala Pro Val Val Glu Ala Val Ser Thr Pro Ser Ala Ala
305                 310                 315                 320
Phe Pro Ser Asp Ala Thr Ala Glu Gln Gly Pro Ile Leu Thr Lys His
                325                 330                 335
Gly Lys Asn Pro Val Met Glu Leu Asn Glu Lys Arg Arg Gly Leu Lys
            340                 345                 350
Tyr Glu Leu Ile Ser Glu Thr Gly Gly Ser His Asp Lys Arg Phe Val
        355                 360                 365
Met Glu Val Glu Val Asp Gly Gln Lys Phe Gln Gly Ala Gly Ser Asn
    370                 375                 380
Lys Lys Val Ala Lys Ala Tyr Ala Ala Leu Ala Ala Leu Glu Lys Leu
385                 390                 395                 400
Phe Pro Asp Thr Pro Leu Ala Leu Asp Ala Asn Lys Lys Lys Arg Ala
                405                 410                 415
Pro Val Pro Val Arg Gly Gly Pro Lys Phe Ala Ala Lys Pro His Asn
            420                 425                 430
Pro Gly Phe Gly Met Gly Gly Pro Met His Asn Glu Val Pro Pro Pro
        435                 440                 445
Pro Asn Leu Arg Gly Arg Gly Arg Gly Gly Ser Ile Arg Gly Arg Gly
    450                 455                 460
Arg Gly Arg Gly Phe Gly Gly Ala Asn His Gly Gly Tyr Met Asn Ala
465                 470                 475                 480
Gly Ala Gly Tyr Gly Ser Tyr Gly Tyr Gly Gly Asn Ser Ala Thr Ala
                485                 490                 495
Gly Tyr Ser Asp Phe Phe Thr Asp Cys Tyr Gly Tyr His Asp Phe Gly
            500                 505                 510

Ser Ser

<210> 25
<211> 422
<212> PRT
<213> Homo sapiens
<400> 25
Met Gly Lys Ala Lys Val Pro Ala Ser Lys Arg Ala Pro Ser Ser Pro
1               5                   10                  15
Val Ala Lys Pro Gly Pro Val Lys Thr Leu Thr Arg Lys Lys Asn Lys
            20                  25                  30
Lys Lys Lys Arg Phe Trp Lys Ser Lys Ala Arg Glu Val Ser Lys Lys
        35                  40                  45
Pro Ala Ser Gly Pro Gly Ala Val Val Arg Pro Pro Lys Ala Pro Glu
    50                  55                  60
Asp Phe Ser Gln Asn Trp Lys Ala Leu Gln Glu Trp Leu Leu Lys Gln
65                  70                  75                  80
Lys Ser Gln Ala Pro Glu Lys Pro Leu Val Ile Ser Gln Met Gly Ser
                85                  90                  95
Lys Lys Lys Pro Lys Ile Ile Gln Gln Asn Lys Lys Glu Thr Ser Pro
            100                 105                 110
Gln Val Lys Gly Glu Glu Met Pro Ala Gly Lys Asp Gln Glu Ala Ser
        115                 120                 125
Arg Gly Ser Val Pro Ser Gly Ser Lys Met Asp Arg Arg Ala Pro Val
    130                 135                 140
Pro Arg Thr Lys Ala Ser Gly Thr Glu His Asn Lys Lys Gly Thr Lys
145                 150                 155                 160
Glu Arg Thr Asn Gly Asp Ile Val Pro Glu Arg Gly Asp Ile Glu His
                165                 170                 175
Lys Lys Arg Lys Ala Lys Glu Ala Ala Pro Ala Pro Pro Thr Glu Glu
            180                 185                 190
Asp Ile Trp Phe Asp Asp Val Asp Pro Ala Asp Ile Glu Ala Ala Ile
        195                 200                 205
Gly Pro Glu Ala Ala Lys Ile Ala Arg Lys Gln Leu Gly Gln Ser Glu
    210                 215                 220
Gly Ser Val Ser Leu Ser Leu Val Lys Glu Gln Ala Phe Gly Gly Leu
225                 230                 235                 240
Thr Arg Ala Leu Ala Leu Asp Cys Glu Met Val Gly Val Gly Pro Lys
                245                 250                 255
Gly Glu Glu Ser Met Ala Ala Arg Val Ser Ile Val Asn Gln Tyr Gly
            260                 265                 270
Lys Cys Val Tyr Asp Lys Tyr Val Lys Pro Thr Glu Pro Val Thr Asp
        275                 280                 285
Tyr Arg Thr Ala Val Ser Gly Ile Arg Pro Glu Asn Leu Lys Gln Gly
    290                 295                 300

Glu Glu Leu Glu Val Val Gln Lys Glu Val Ala Glu Met Leu Lys Gly
305                 310                 315                 320
Arg Ile Leu Val Gly His Ala Leu His Asn Asp Leu Lys Val Leu Phe
                325                 330                 335
Leu Asp His Pro Lys Lys Lys Ile Arg Asp Thr Gln Lys Tyr Lys Pro
            340                 345                 350
Phe Lys Ser Gln Val Lys Ser Gly Arg Pro Ser Leu Arg Leu Leu Ser
        355                 360                 365
Glu Lys Ile Leu Gly Leu Gln Val Gln Gln Ala Glu His Cys Ser Ile
    370                 375                 380
Gln Asp Ala Gln Ala Ala Met Arg Leu Tyr Val Met Val Lys Lys Glu
385                 390                 395                 400
Trp Glu Ser Met Ala Arg Asp Arg Arg Pro Leu Leu Thr Ala Pro Asp
                405                 410                 415
His Cys Ser Asp Asp Ala
            420

<210> 26
<211> 422
<212> PRT
<213> Homo sapiens
<400> 26
Met Gly Lys Ala Lys Val Pro Ala Ser Lys Arg Ala Pro Ser Ser Pro
 1               5                  10                  15
Val Ala Lys Pro Gly Pro Val Lys Thr Leu Thr Arg Lys Lys Asn Lys
            20                  25                  30
Lys Lys Lys Arg Phe Trp Lys Ser Lys Ala Arg Glu Val Ser Lys Lys
        35                  40                  45
Pro Ala Ser Gly Pro Gly Ala Val Val Arg Pro Pro Lys Ala Pro Glu
    50                  55                  60
Asp Phe Ser Gln Asn Trp Lys Ala Leu Gln Glu Trp Leu Leu Lys Gln
65                  70                  75                  80
Lys Ser Gln Ala Pro Glu Lys Pro Leu Val Ile Ser Gln Met Gly Ser
                85                  90                  95
Lys Lys Lys Pro Lys Ile Ile Gln Gln Asn Lys Lys Glu Thr Ser Pro
            100                 105                 110
Gln Val Lys Gly Glu Glu Met Pro Ala Gly Lys Asp Gln Glu Ala Ser
        115                 120                 125
Arg Gly Ser Val Pro Ser Gly Ser Lys Met Asp Arg Arg Ala Pro Val
    130                 135                 140
Pro Arg Thr Lys Ala Ser Gly Thr Glu His Asn Lys Lys Gly Thr Lys
145                 150                 155                 160
Glu Arg Thr Asn Gly Asp Ile Val Pro Glu Arg Gly Asp Ile Glu His
                165                 170                 175
Lys Lys Arg Lys Ala Lys Glu Ala Ala Pro Ala Pro Pro Thr Glu Glu
            180                 185                 190

Asp Ile Trp Phe Asp Asp Val Asp Pro Pro Asp Ile Glu Ala Ala Ile
        195                 200                 205
Gly Pro Glu Ala Ala Lys Ile Ala Arg Lys Gln Leu Gly Gln Ser Glu
    210                 215                 220
Gly Ser Val Ser Leu Ser Leu Val Lys Glu Gln Ala Phe Gly Gly Leu
225                 230                 235                 240
Thr Arg Ala Leu Ala Leu Asp Cys Glu Met Val Gly Val Gly Pro Lys
                245                 250                 255
Gly Glu Glu Ser Met Ala Ala Arg Val Ser Ile Val Asn Gln Tyr Gly
            260                 265                 270
Lys Cys Val Tyr Asp Lys Tyr Val Lys Pro Thr Glu Pro Val Thr Asp
        275                 280                 285
Tyr Arg Thr Ala Val Ser Gly Ile Arg Pro Glu Asn Leu Lys Gln Gly
    290                 295                 300
Glu Glu Leu Glu Val Val Gln Lys Glu Val Ala Glu Met Leu Lys Gly
305                 310                 315                 320
Arg Ile Leu Val Gly His Ala Leu His Asn Asp Leu Lys Val Leu Phe
                325                 330                 335
Leu Asp His Pro Lys Lys Lys Ile Arg Asp Thr Gln Lys Tyr Lys Pro
            340                 345                 350
Phe Lys Ser Gln Val Lys Ser Gly Arg Pro Ser Leu Arg Leu Leu Ser
        355                 360                 365
Glu Lys Ile Leu Gly Leu Gln Val Gln Gln Ala Glu His Cys Ser Ile
    370                 375                 380
Gln Asp Ala Gln Ala Ala Met Arg Leu Tyr Val Met Val Lys Lys Glu
385                 390                 395                 400
Trp Glu Ser Met Ala Arg Asp Arg Arg Pro Leu Leu Thr Ala Pro Asp
                405                 410                 415
His Cys Ser Asp Asp Ala
            420

<210> 27
<211> 422
<212> PRT
<213> Homo sapiens
<400> 27
Met Gly Lys Ala Lys Val Pro Ala Ser Lys Arg Ala Pro Ser Ser Pro
  1               5                  10                  15
Val Ala Lys Pro Gly Pro Val Lys Thr Leu Thr Arg Lys Lys Asn Lys
             20                  25                  30
Lys Lys Lys Arg Phe Trp Lys Ser Lys Ala Arg Glu Val Ser Lys Lys
         35                  40                  45
Pro Ala Ser Gly Pro Gly Ala Val Val Arg Pro Pro Lys Ala Pro Glu
     50                  55                  60
Asp Phe Ser Gln Asn Trp Lys Ala Leu Gln Glu Trp Leu Leu Lys Gln
 65                  70                  75                  80

Lys Ser Gln Ala Pro Glu Lys Pro Leu Val Ile Ser Gln Met Gly Ser
                85                  90                  95
Lys Lys Lys Pro Lys Ile Ile Gln Gln Asn Lys Lys Glu Thr Ser Pro
            100                 105                 110
Gln Val Lys Gly Glu Glu Met Pro Ala Gly Lys Asp Gln Glu Ala Ser
        115                 120                 125
Arg Gly Ser Val Pro Ser Gly Ser Lys Met Asp Arg Arg Ala Pro Val
    130                 135                 140
Pro Arg Thr Lys Ala Ser Gly Thr Glu His Asn Lys Lys Gly Thr Lys
145                 150                 155                 160
Glu Arg Thr Asn Gly Asp Ile Val Pro Glu Arg Gly Asp Ile Glu His
                165                 170                 175
Lys Lys Arg Lys Ala Lys Glu Ala Ala Pro Ala Pro Pro Thr Glu Glu
            180                 185                 190
Asp Ile Trp Phe Asp Asp Val Asp Pro Pro Asp Ile Glu Ala Ala Ile
        195                 200                 205
Gly Pro Glu Ala Ala Lys Ile Ala Arg Lys Gln Leu Gly Gln Ser Glu
    210                 215                 220
Gly Ser Val Ser Leu Ser Leu Val Lys Glu Gln Ala Phe Gly Gly Leu
225                 230                 235                 240
Thr Arg Ala Leu Ala Leu Asp Cys Glu Met Val Gly Val Gly Pro Lys
                245                 250                 255
Gly Glu Glu Ser Met Ala Ala Arg Val Ser Ile Val Asn Gln Tyr Gly
            260                 265                 270
Lys Cys Val Tyr Asp Lys Tyr Val Lys Pro Thr Glu Pro Val Thr Asp
        275                 280                 285
Tyr Arg Thr Ala Val Ser Gly Ile Arg Pro Glu Asn Leu Lys Gln Gly
    290                 295                 300
Glu Glu Leu Glu Val Val Gln Lys Glu Val Ala Glu Met Leu Lys Gly
305                 310                 315                 320
Arg Ile Leu Val Gly His Ala Leu His Asn Asp Leu Lys Val Leu Phe
                325                 330                 335
Leu Asp His Pro Lys Lys Lys Ile Arg Asp Thr Gln Lys Tyr Lys Pro
            340                 345                 350
Phe Lys Ser Gln Val Lys Ser Gly Arg Pro Ser Leu Arg Leu Leu Ser
        355                 360                 365
Glu Lys Ile Leu Gly Leu Gln Val Gln Gln Ala Glu His Cys Ser Ile
    370                 375                 380
Gln Asp Ala Gln Ala Ala Met Arg Leu Tyr Val Met Val Lys Lys Glu
385                 390                 395                 400
Trp Glu Ser Met Ala Arg Asp Arg Arg Pro Leu Leu Thr Ala Pro Asp
                405                 410                 415
His Cys Ser Asp Asp Ala
            420

<210> 28

<211> 422
<212> PRT
<213> Homo sapiens
<400> 28
Met Gly Lys Ala Lys Val Pro Ala Ser Lys Arg Ala Pro Ser Ser Pro
1               5                   10                  15
Val Ala Lys Pro Gly Pro Val Lys Thr Leu Thr Arg Lys Lys Asn Lys
            20                  25                  30
Lys Lys Lys Arg Phe Trp Lys Ser Lys Ala Arg Glu Val Ser Lys Lys
        35                  40                  45
Pro Ala Ser Gly Pro Gly Ala Val Val Arg Pro Pro Lys Ala Pro Glu
    50                  55                  60
Asp Phe Ser Gln Asn Trp Lys Ala Leu Gln Glu Trp Leu Leu Lys Gln
65                  70                  75                  80
Lys Ser Gln Ala Pro Glu Lys Pro Leu Val Ile Ser Gln Met Gly Ser
                85                  90                  95
Lys Lys Lys Pro Lys Ile Ile Gln Gln Asn Lys Lys Glu Thr Ser Pro
            100                 105                 110
Gln Val Lys Gly Glu Glu Met Pro Ala Gly Lys Asp Gln Glu Ala Ser
        115                 120                 125
Arg Gly Ser Val Pro Ser Gly Ser Lys Met Asp Arg Arg Ala Pro Val
    130                 135                 140
Pro Arg Thr Lys Ala Ser Gly Thr Glu His Asn Lys Lys Gly Thr Lys
145                 150                 155                 160
Glu Arg Thr Asn Gly Asp Ile Val Pro Glu Arg Gly Asp Ile Glu His
                165                 170                 175
Lys Lys Arg Lys Ala Lys Glu Ala Ala Pro Ala Pro Pro Thr Glu Glu
            180                 185                 190
Asp Ile Trp Phe Asp Asp Val Asp Pro Ala Asp Ile Glu Ala Ala Ile
        195                 200                 205
Gly Pro Glu Ala Ala Lys Ile Ala Arg Lys Gln Leu Gly Gln Ser Glu
    210                 215                 220
Gly Ser Val Ser Leu Ser Leu Val Lys Glu Gln Ala Phe Gly Gly Leu
225                 230                 235                 240
Thr Arg Ala Leu Ala Leu Asp Cys Glu Met Val Gly Val Gly Pro Lys
                245                 250                 255
Gly Glu Glu Ser Met Ala Ala Arg Val Ser Ile Val Asn Gln Tyr Gly
            260                 265                 270
Lys Cys Val Tyr Asp Lys Tyr Val Lys Pro Thr Glu Pro Val Thr Asp
        275                 280                 285
Tyr Arg Thr Ala Val Ser Gly Ile Arg Pro Glu Asn Leu Lys Gln Gly
    290                 295                 300
Glu Glu Leu Glu Val Val Gln Lys Glu Val Ala Glu Met Leu Lys Gly
305                 310                 315                 320
Arg Ile Leu Val Gly His Ala Leu His Asn Asp Leu Lys Val Leu Phe
                325                 330                 335

Leu Asp His Pro Lys Lys Lys Ile Arg Asp Thr Gln Lys Tyr Lys Pro
            340                 345                 350
Phe Lys Ser Gln Val Lys Ser Gly Arg Pro Ser Leu Arg Leu Leu Ser
        355                 360                 365
Glu Lys Ile Leu Gly Leu Gln Val Gln Gln Ala Glu His Cys Ser Ile
    370                 375                 380
Gln Asp Ala Gln Ala Ala Met Arg Leu Tyr Val Met Val Lys Lys Glu
385                 390                 395                 400
Trp Glu Ser Met Ala Arg Asp Arg Arg Pro Leu Leu Thr Ala Pro Asp
                405                 410                 415
His Cys Ser Asp Asp Ala
            420

<210> 29
<211> 353
<212> PRT
<213> Homo sapiens
<400> 29
Met Ser Thr Leu Leu Leu Asn Leu Asp Phe Gly Glu Pro Pro Pro Lys
1               5                   10                  15
Lys Ala Leu Glu Gly Asn Ala Lys His Arg Asn Phe Val Lys Lys Arg
            20                  25                  30
Arg Leu Leu Glu Arg Arg Gly Phe Leu Ser Lys Lys Asn Gln Pro Pro
        35                  40                  45
Ser Lys Ala Pro Lys Leu His Ser Glu Pro Ser Lys Lys Gly Glu Thr
    50                  55                  60
Pro Thr Val Asp Gly Thr Trp Lys Thr Pro Ser Phe Pro Lys Lys Lys
65                  70                  75                  80
Thr Ala Ala Ser Ser Asn Gly Ser Gly Gln Pro Leu Asp Lys Lys Ala
                85                  90                  95
Ala Val Ser Trp Leu Thr Pro Ala Pro Ser Lys Lys Ala Asp Ser Val
            100                 105                 110
Ala Ala Lys Val Asp Leu Leu Gly Glu Phe Gln Ser Ala Leu Pro Lys
        115                 120                 125
Ile Asn Ser His Pro Thr Arg Ser Gln Lys Lys Ser Ser Gln Lys Lys
    130                 135                 140
Ser Ser Lys Lys Asn His Pro Gln Lys Asn Ala Pro Gln Asn Ser Thr
145                 150                 155                 160
Gln Ala His Ser Glu Asn Lys Cys Ser Gly Ala Ser Gln Lys Leu Pro
                165                 170                 175
Arg Lys Met Val Ala Ile Asp Cys Glu Met Val Gly Thr Gly Pro Lys
            180                 185                 190
Gly His Val Ser Ser Leu Ala Arg Cys Ser Ile Val Asn Tyr Asn Gly
        195                 200                 205
Asp Val Leu Tyr Asp Glu Tyr Ile Leu Pro Pro Cys His Ile Val Asp
    210                 215                 220

Tyr Arg Thr Arg Trp Ser Gly Ile Arg Lys Gln His Met Val Asn Ala
225                 230                 235                 240
Thr Pro Phe Lys Ile Ala Arg Gly Gln Ile Leu Lys Ile Leu Thr Gly
                245                 250                 255
Lys Ile Val Val Gly His Ala Ile His Asn Asp Phe Lys Ala Leu Gln
            260                 265                 270
Tyr Phe His Pro Lys Ser Leu Thr Arg Asp Thr Ser His Ile Pro Pro
        275                 280                 285
Leu Asn Arg Lys Ala Asp Cys Pro Glu Asn Ala Thr Met Ser Leu Lys
    290                 295                 300
His Leu Thr Lys Lys Leu Leu Asn Arg Asp Ile Gln Val Gly Lys Ser
305                 310                 315                 320
Gly His Ser Ser Val Glu Asp Ala Gln Ala Thr Met Glu Leu Tyr Lys
                325                 330                 335
Leu Val Glu Val Glu Trp Glu Glu His Leu Ala Arg Asn Pro Pro Thr
            340                 345                 350
Asp

<210> 30
<211> 209
<212> PRT
<213> Homo sapiens
<400> 30
Met Val Gly Thr Gly Pro Arg Gly Arg Val Ser Glu Leu Ala Arg Cys
1               5                   10                  15
Ser Ile Val Ser Tyr His Gly Asp Val Leu Tyr Asp Lys Tyr Ile Arg
            20                  25                  30
Pro Glu Met Pro Ile Ala Asp Tyr Arg Thr Arg Trp Ser Gly Ile Thr
        35                  40                  45
Arg Gln His Met Arg Lys Ala Val Pro Phe Gln Val Ala Gln Lys Glu
    50                  55                  60
Ile Leu Lys Leu Leu Lys Gly Lys Val Val Val Gly His Ala Leu His
65                  70                  75                  80
Asn Asp Phe Gln Ala Leu Lys Tyr Val His Pro Arg Ser Gln Thr Arg
                85                  90                  95
Asp Thr Thr Tyr Val Pro Asn Phe Leu Ser Glu Pro Gly Leu His Thr
            100                 105                 110
Arg Ala Arg Val Ser Leu Lys Asp Leu Ala Leu Gln Leu Leu His Lys
        115                 120                 125
Lys Ile Gln Val Gly Gln His Gly His Ser Ser Val Glu Asp Ala Thr
    130                 135                 140
Thr Ala Met Glu Leu Tyr Arg Leu Val Glu Val Gln Trp Glu Gln Gln
145                 150                 155                 160
Glu Ala Arg Ser Leu Trp Thr Cys Pro Glu Asp Arg Glu Pro Asp Ser
                165                 170                 175

Ser Thr Asp Met Glu Gln Tyr Met Glu Asp Gln Tyr Trp Pro Asp Asp
            180                 185                 190
Leu Ala His Gly Ser Arg Gly Gly Ala Arg Glu Ala Gln Asp Arg Arg
        195                 200                 205
Asn

<210> 31
<211> 476
<212> DNA
<213> Homo sapiens
<400> 31
tctggctcac ctggaggaag aggtccacga caaggtggtt gcccgggacc tggttggagc 60
catgttgagc ccactgccgc agccacgccc ctctgccccc caggtgctgg cccaccccgt 120
tcttttggag cagancaagc aactccagtt cttccaggac gtcagtgact ggctggagaa 180
ggagtccgag caggagcccc tggtgagggc actggaggcg ggaggctgcg cagtggtccg 240
ggacaactgg cacgagcaca tctccatgcc gctgcagaca gatctgagaa agttccggtc 300
ctataagggg acatcagtgc gagacctgct ccgtgctgtg aggaacaaga agcaccacta 360
cagggagctc ccagttgagg tgcgacaggc attcggccaa gtccctgatg gcttcgtcca 420
gtacttcaca aaccgctttc caanggctgc tcntcaaang caccgagcca tgagga   476